(19) Europäisches Patentamt
European Patent Office
Office européen des brevets

(11) EP 4 640 717 A1

# (12) EUROPEAN PATENT APPLICATION
published in accordance with Art. 153(4) EPC

(43) Date of publication:
**29.10.2025 Bulletin 2025/44**

(21) Application number: **23910847.5**

(22) Date of filing: **28.12.2023**

(51) International Patent Classification (IPC):
***C08B 37/08*** *(2006.01)* ***A61L 27/52*** *(2006.01)*

(52) Cooperative Patent Classification (CPC):
Y02P 20/55

(86) International application number:
**PCT/CN2023/142840**

(87) International publication number:
**WO 2024/140931 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.12.2022 CN 202211738168**

(71) Applicant: **SHANGHAI JIAOTONG UNIVERSITY**
**Minhang District**
**Shanghai 200240 (CN)**

(72) Inventors:
- **LIN, Qiuning**
  **Shanghai 200240 (CN)**
- **CHEN, Ting**
  **Shanghai 200240 (CN)**
- **BAO, Bingkun**
  **Shanghai 200240 (CN)**
- **XIAO, Chaonan**
  **Shanghai 200240 (CN)**
- **ZHU, Linyong**
  **Shanghai 200240 (CN)**

(74) Representative: **Sun, Yiming**
**HUASUN Patent- und Rechtsanwälte**
**Friedrichstraße 33**
**80801 München (DE)**

## (54) POLYSACCHARIDE-BASED MACROMOLECULE CROSS-LINKING AGENT, POLYSACCHARIDE-BASED BIOLOGICAL MATERIAL, AND PREPARATION METHOD THEREFOR AND USE THEREOF

(57) The present invention relates to a polysaccharide-based macromolecule cross-linking agent, a polysaccharide-based biological material, and a preparation method therefor and the use thereof. The polysaccharide-based macromolecule cross-linking agent of the present invention is a polysaccharide-based macromolecule cross-linking agent modified with an o-phthalaldehyde group, and the polysaccharide-based polymer cross-linking agent does not depend on an artificially synthesized hydrophilic macromolecule skeleton and a complex chemical synthesis step. The polysaccharide-based macromolecule cross-linking agent provided in the present invention, together with a water-soluble small molecule or macromolecule containing one or more groups of a primary amine, hydrazine, hydrazide, hydroxylamine or sulfhydryl, forms a polysaccharide-based two-component hydrogel material, which has the advantages of injectability, low swelling and strong tissue adhesion. In addition, the polysaccharide-based two-component hydrogel material provided in the present invention can be further prepared into a polysaccharide-based biological material in the form of a film, powder, sponge, etc., can be used as a tissue adhesive, a sealant, a hemostatic agent, a tissue engineering material, etc., and has Wide biomedical application prospects.

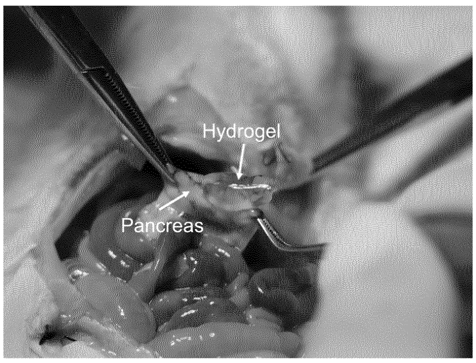


Fig. 5

EP 4 640 717 A1

## Description

### Technical Field

The invention belongs to the field of biomaterials, and specially relates to polysaccharide-based polymer crosslinkers, polysaccharide-based biomaterials and preparation methods and applications.

### Related Art

Hydrogel is a highly water-containing polymer material with three-dimensional crosslinked network structure with adjustable physicochemical properties, and its high-water content, biocompatibility, and multifunctionality have led to a wide range of applications in many biomedical fields. Among them, dual-component hydrogels can be used as hemostatic agents, tissue adhesives and sealants in the clinic by taking advantage of their in-situ molding and curing properties. For example, the DuraSeal gel produced by Confluent Surgical (U.S.) and the CoSeal gel produced by Baxter (U.S.) take polyethylene glycol as a backbone, and achieve crosslinking based on the reaction between amino or sulfhydryl groups and active esters on the end groups, and at the same time, through the reaction between carboxylic acid active esters and amino and other active groups on the surface of the tissues, adhere to the tissues, and implement closure of traumatized tissues; and for example, Chinese patents CN111440310A, CN111440334A, CN 111574756A, CN 111621038 A, CN113509591A, CN113694249A, and CN114767920A disclose a series of polyethylene glycol crosslinkers modified by o-phthalaldehyde groups, which are prepared by crosslinking o-phthalaldehyde with amino-containing polymers. A dual-component hydrogel prepared by crosslinking o-phthalaldehyde with an amino-containing polymer can be used as a tissue sealant. However, due to the extreme hydrophilicity of the polyethylene glycol skeleton, the swelling rate of such hydrogels is generally high (>100%), and their high swelling not only greatly reduces the mechanical properties of the hydrogel, but also the swelling of the volume causes compression of the surrounding tissues, and at the same time the gels have the risk of detachment, which makes the safety of such hydrogels hazardous in clinical applications. For example, polyethylene glycol-based sealants, represented by DuraSeal and CoSeal gels, are commonly used in neurosurgery to prevent cerebrospinal fluid leakage by sealing the dura mater after suturing, however, in actual clinical applications, several adverse events resulting in tetraplegia were caused due to the compression of nerves by volume swelling of the hydrogel after absorbing body fluids (D Thavarajah, P De Lacy, R Hussain, RM Redfern, Spine. 2010, 35, 25-26.).

To address the swelling problem, Chinese Patent CN114907558A discloses a method of preparing polyethylene glycol-based low-swelling hydrogels by reducing the hydrophilicity of polyethylene glycol polymer backbones through hydrophobic modification of the polyethylene glycol polymer backbone; however, the method is based on a complex modification of the polyethylene glycol backbone, which involves a multi-step chemical synthesis.

### SUMMARY OF INVENTION

To solve the problem of high swelling ratio prevalent in polyethylene glycol-based hydrogels in the prior art, the present invention provides polysaccharide-based polymer crosslinkers, polysaccharide-based biomaterials, and preparation methods and applications.

Specifically, the invention provides polysaccharide-based polymer crosslinkers, preparation methods of polysaccharide-based polymer crosslinkers, polysaccharide-based biomaterials, preparation methods of polysaccharide-based biomaterials, and applications of polysaccharide-based biomaterials.

The objectives of the invention can be realized by the following technical solutions:

The first objective of the invention is to provide a class of polysaccharide-based polymer crosslinkers.

The polysaccharide-based polymer crosslinker is a polysaccharide-based polymer crosslinker modified with o-phthalaldehyde groups, and the structure is shown in Formula 1:

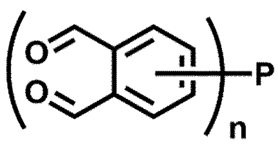

Formula 1

Wherein, P is a natural polysaccharide macromolecule or a modifier or degradation thereof, and P is selected from one or more of hyaluronic acid, cellulose, cellulose derivatives, alginate, dextran, agarose, heparin, chondroitin

sulfate, carrageenan, astragalus gum, xanthan gum, gellan gum, guar gum, gum arabic, acacia bean gum, starch, starch hydrolysate, or starch derivatives;

n ≥ 2; which means that the average number of o-phthalaldehyde functional groups on a single polysaccharide polymer chain is greater than or equal to 2;

The o-phthalaldehyde group is connected by a covalent bond to P.

In some embodiments of the invention, P is selected from hyaluronic acid, cellulose derivatives, alginate, heparin, chondroitin sulfate, xanthan gum, gellan gum, starch, or hydrolysis products or derivatives thereof.
The cellulose derivative is selected from methyl cellulose, carboxymethyl cellulose, hydroxyethyl cellulose, hydroxypropyl cellulose or hydroxypropylmethyl cellulose;

Preferably, cellulose derivative is selected from carboxymethyl cellulose;

The starch derivative is selected from oxidized starch, esterified starch, etherified starch or alkylated starch;

Preferably, starch derivative is selected from carboxymethyl starch.

In some specific embodiments of the invention, the o-phthalaldehyde group-modified polysaccharide-based polymer crosslinker is selected from one of the following structures:

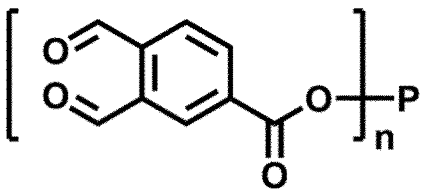
,
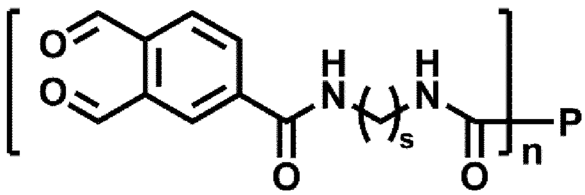
,

Formula 1-1 Formula 1-2

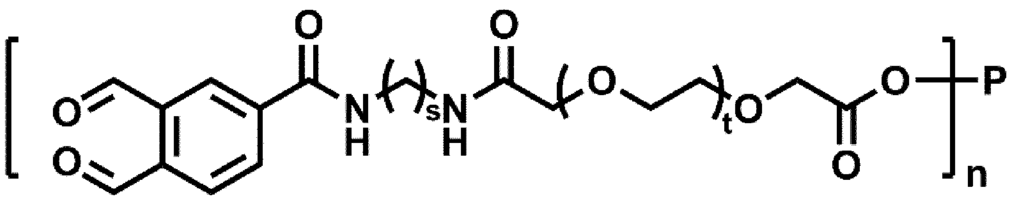

Formula 1-3

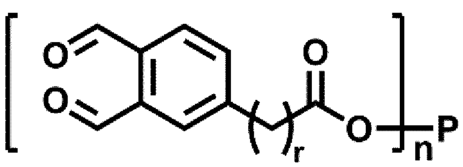
,
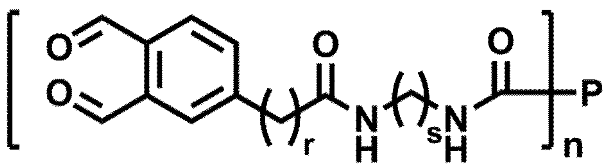
,

Formula 1-4 Formula 1-5

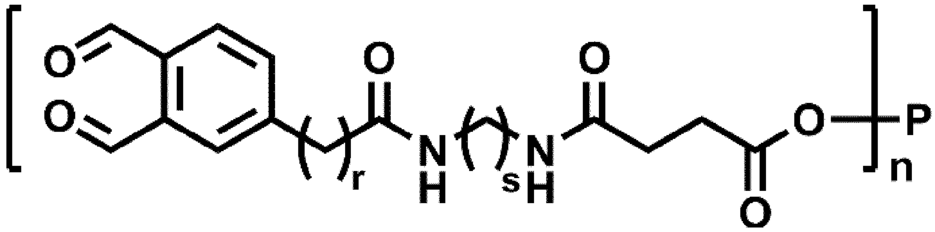,

Formula 1-6

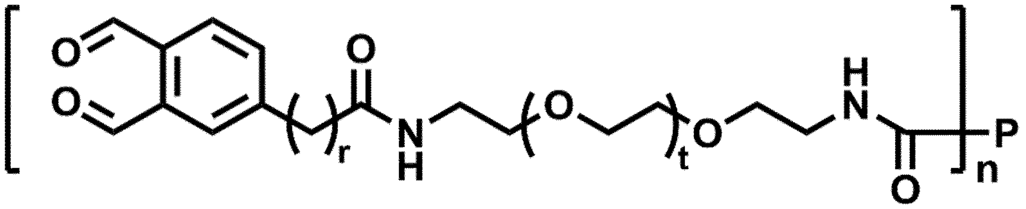,

Formula 1-7

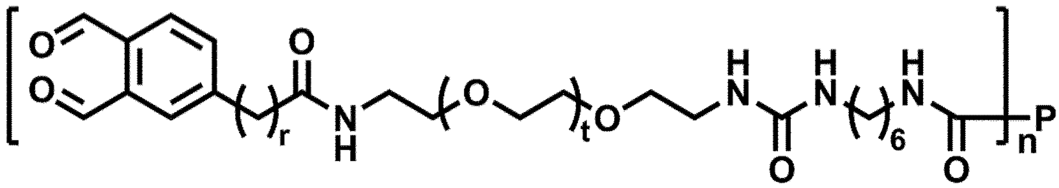,

Formula 1-8

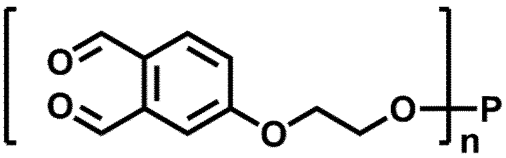, 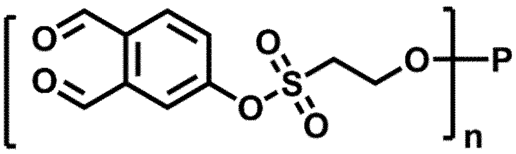,

Formula 1-9 Formula 1-10

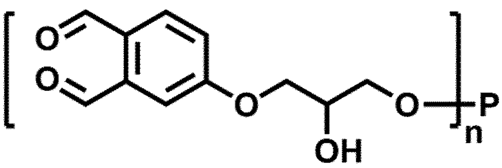, 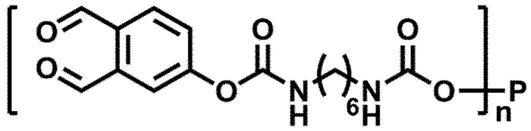

Formula 1-11 Formula 1-12

Wherein, $1 \leq r \leq 20$, $1 \leq s \leq 20$, $1 \leq t \leq 50$, $n \geq 2$. Preferably, $1 \leq r \leq 6$, $1 \leq s \leq 10$, $1 \leq t \leq 30$, $n \geq 2$.

The second objective of the invention is to provide a method of preparing the polysaccharide-based polymer crosslinkers.

The polysaccharide-based polymer crosslinker is prepared as follows:

The aldehyde pre-protected o-phthalaldehyde precursor derivative is covalently bonded to an active group on the polysaccharide polymer to obtain a polysaccharide polymer modified by the o-phthalaldehyde precursor derivative,

and the aldehyde group of the polysaccharide polymer modified by the o-phthalaldehyde precursor derivative is subsequently de-protected to obtain the polysaccharide polymer crosslinker modified by the o-phthalaldehyde group.

The aldehyde pre-protected o-phthalaldehyde precursor derivative comprises an aldehyde pre-protected o-phthalaldehyde moiety and a substituent R that can be linked to a reactive group on the polysaccharide macromolecule, with a structure as shown in Formula 2:

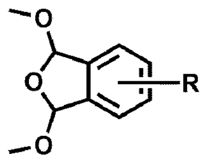

Formula 2

Wherein, R is selected from a carboxyl substituent, a vinyl sulfone substituent, an epoxy substituent, a haloalkane substituent, an isocyanate substituent, or an amine substituent;

R is directly connected to the benzene ring or connected to the benzene ring through single or multiple alkylidene chains, alkoxy chains. These multiple alkylidene chains, alkoxy chains are connected to each other through ether, amide, ester, carbamate or urea bonds.

In some specific embodiments of the invention, the aldehyde pre-protected o-phthalaldehyde precursor derivative is selected from one of the following compounds:

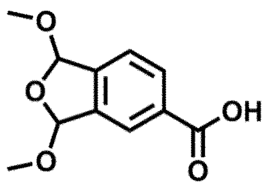 , 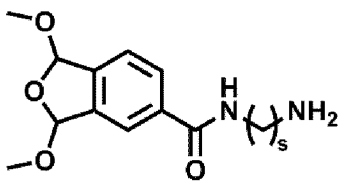 ,

Compound 1 Compound 2

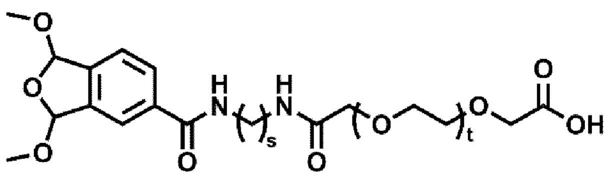 , 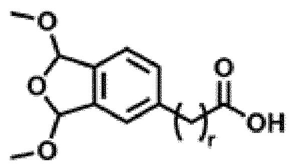 ,

Compound 3 Compound 4

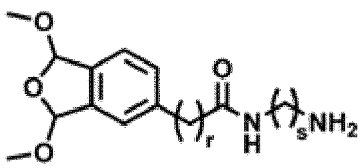 , 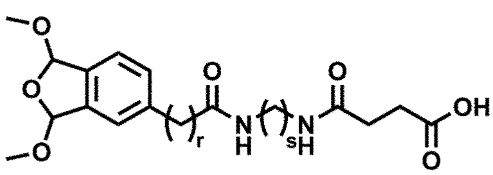 ,

Compound 5 Compound 6

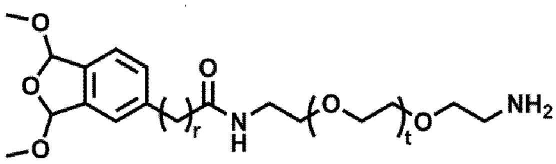

Compound 7

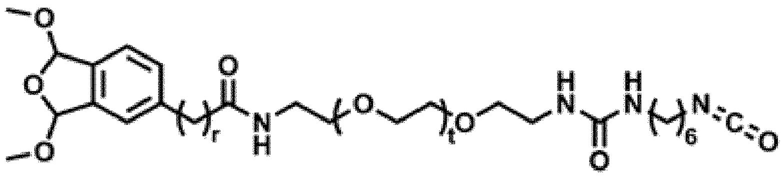

Compound 8

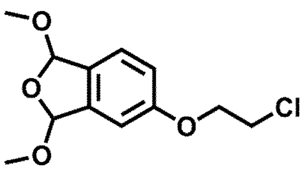

,

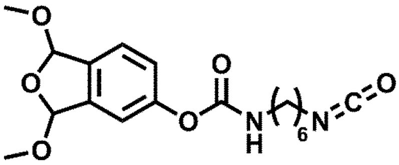

,

Compound 9 Compound 10

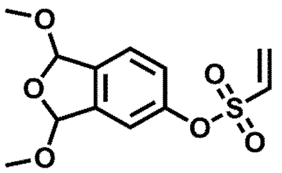

,

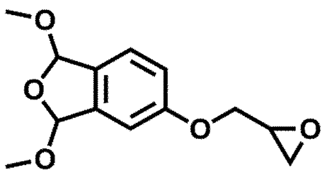

Compound 11 Compound 12

Wherein, $1 \le r \le 20$, $1 \le s \le 20$, $1 \le t \le 50$, $n \ge 2$. Preferably, $1 \le r \le 6$, $1 \le s \le 10$, $1 \le t \le 30$, $n \ge 2$.

The reactive groups of the polysaccharide polymer can be either the polysaccharide polymer's own or reactive groups after the polysaccharide polymer has been modified or degraded.

In some embodiments of the invention, the reactive groups of the polysaccharide polymer are selected from hydroxyl and carboxyl groups.

The aldehyde group pre-protected o-phthalaldehyde precursor derivative is covalently bonded to the active group on the polysaccharide polymer in a manner selected from ether bond, amide bond, ester bond, carbamate bond or urea bond connection, preferably ether bond, amide bond, ester bond, carbamate bond connection.

When the active group of the polysaccharide polymer is selected from hydroxyl, the covalent bonding is based on ether bonding between vinyl sulfone substituents, epoxy substituents, or haloalkane substituents on the precursor derivatives of o-phthalaldehyde and hydroxyl groups on the polysaccharide polymer, on ester bonding between carboxylic acid substituents on o-phthalaldehyde precursor derivatives and hydroxyl groups on the polysaccharide polymer, on carbamate bonding between isocyanate substituents on o-phthalaldehyde precursor derivatives and hydroxyl groups on the polysaccharide polymer.

When the active group of the polysaccharide macromolecule is selected from a carboxyl group, the covalent bonding connection is based on an amide bonding connection between an amine group-like substituent on a precursor derivative of o-phthalaldehyde and a carboxyl group on the polysaccharide polymer.

In some specific embodiments of the invention, the covalent bonding is preferably based on an ether bonding

between a vinyl sulfone substituent on the precursor derivative of o-phthalaldehyde and a hydroxyl group on the polysaccharide polymer, an ether bonding between an epoxy substituent on the precursor derivative of o-phthalaldehyde and a hydroxyl group on the polysaccharide polymer, an ester bonding between a carboxylic acid substituent on the precursor derivative of o-phthalaldehyde and a hydroxyl group on the polysaccharide polymer, and an amide bonding between an amine group substituent on the precursor derivative of o-phthalaldehyde and a carboxyl group on the polysaccharide polymer. hydroxyl group on the polysaccharide macromolecule, and based on an amide bond connection between an amine substituent on the precursor derivative of o-phthalaldehyde and a carboxyl group on the polysaccharide macromolecule.

Deprotection of the aldehyde groups of polysaccharide polymers modified with o-phthalaldehyde precursor derivatives refers to the deprotection of o-phthalaldehyde functional groups on polysaccharide polymers with the aldehyde groups attached to the polysaccharide polymers being pre-protected to obtain o-phthalaldehyde group-modified o-polysaccharide-based polymer cross-linking agents under acidic conditions.

The structures of the o-phthalaldehyde group-modified polysaccharide-based polymer crosslinkers provided in Formula 1-1 to Formula 1-12 of the first objective of the invention are merely exemplary illustrations of the o-phthalaldehyde group-modified polysaccharide-based polymer crosslinkers that can be obtained by certain preparation methods of the aldehyde pre-protected o-phthalaldehyde precursor derivative and polysaccharide polymer involved above. The person skilled in the art can think of other aldehyde pre-protected o-phthalaldehyde precursor derivative structures that can be used to realize the o-phthalaldehyde group-modified polysaccharide-based polymer crosslinker according to the contents of the present application.

The third objective of the invention is to provide a method of preparing a polysaccharide-based dual-component hydrogel: component A and component B are dissolved in a solvent to obtain a component A solution and a component B solution, and the component A solution and the component B solution are the dual components of the polysaccharide-based dual-component hydrogel; and mixing of the component A solution and the component B solution results in the polysaccharide-based dual-component hydrogel;

Wherein, the component A is a polysaccharide-based polymer crosslinker modified with an o-phthalaldehyde group as shown in Formula 1, $n \geq 2$;

The component B is a water-soluble small molecule, a water-soluble synthetic macromolecule, a water-soluble natural macromolecule containing one or more of primary amine, biphenylamine, hydrazide, hydroxylamine or sulfhydryl groups, and the number of primary amine, biphenylamine, hydrazide, hydroxylamine or sulfhydryl functional groups contained on a single molecule is not less than 2;

In some embodiments of the invention, preferably, the component B is selected from one or more of the following substances: polyethylene glycol derivatives, polyethyleneimines, polyamino acids, proteins, protein modifiers, protein degradates, polysaccharides, polysaccharide modifiers or polysaccharide degradates; and the molecular structure of the substance selected for the component B contains one or more of a primary amine, a bipyramid, a hydrazide, a hydroxyl amine, or a sulfhydryl groups, and the number of primary amine, biphenylamine, hydrazide, hydroxylamine or sulfhydryl functional groups contained on a single molecule is not less than 2;

Preferably, the polyethylene glycol derivative is a polyethylene glycol derivative modified with a primary amine, a biphenylamine, a hydrazide, a hydroxylamine or a sulfhydryl group;

The proteins comprise collagen, serum proteins, fibrinogen and fibronectin, and the protein degradates comprise gelatin or peptides;

The polysaccharides, polysaccharide modifiers or polysaccharide degraders are selected from: chitosan and chitosan modifiers and chitosan degraders; tertiary amine, hydrazide, hydroxyl amine or sulfhydryl modified hyaluronic acid, alginic acid, chondroitin sulphate, heparin, cellulose, chitin and their respective modifiers and degraders.

Further preferably, the component B is selected from amine-modified polyethylene glycol derivatives; hydrazide-modified hyaluronic acid; collagen; serum proteins; gelatin; polypeptides; polyamino acids; chitosan.

In some embodiments of the invention, the solvent is selected from water, saline, buffer solution, decellularized substrate, or cell culture medium solution.

In some embodiments of the invention, preferably, in the component A solution, the solid content of component A is 0.1-40 wt%, preferably 0.5-20 wt%, and further preferably 0.5-10 wt%; and in the component B solution, the solid content of component B is 0.1-40 wt%, preferably 0.5-20 wt%, and further preferably 0.5-10 wt%. Among them, increasing the

proportion of component A in the polysaccharide-based dual-component hydrogel can effectively improve the tissue adhesion properties and hemostatic properties of the hydrogel.

In some embodiments of the invention, the preparation temperature of the hydrogel prepared by mixing the Component A solution and the Component B solution is 0-80°C; the preparation pH is 1-12.

The fourth objective of the invention is to provide polysaccharide-based dual-component hydrogels obtained by preparation based on the method of the third objective of the invention.

The fifth objective of the invention is to provide a variety of polysaccharide-based biomaterials.

In some embodiments of the invention, the polysaccharide-based biomaterial, is a polysaccharide-based membrane material made by drying a polysaccharide-based dual-component hydrogel provided in the fourth objective of the invention.

In some embodiments of the invention, the drying method comprises natural drying and drying.

Preferably, the drying condition is drying at 30°C for 12 hours.

In some embodiments of the invention, the polysaccharide-based biomaterial, is a polysaccharide-based powder material obtained by mechanical ball milling of the polysaccharide-based membrane material. The mechanical ball milling means that the polysaccharide-based membrane material is loaded into a ball mill, and the material is between strongly stirred milling balls, and is subjected to repeated effects of impact, grinding force, shear force and pressure, so that it is continuously deformed and broken, and a micro-fine powder is obtained. In order to reduce the self-adhesion between particles and prevent their agglomeration, liquid surfactants and lubricants, such as ethanol, can be added. It is required that the liquid additives shall not corrode the particles or have chemical reaction with them.

In other embodiments of the invention, the polysaccharide-based biomaterial, is a polysaccharide-based powder material obtained by mixing the component A solution and the component B solution provided in the third objective of the invention and preparing microgel particles by emulsification, microfluidic methods and mechanical pulverization, etc., and further obtained by drying and sieving.

Preferably, the preparation method of the above microgel particles is the mechanical pulverization method: the polysaccharide-based hydrogel obtained by mixing the component A solution and the component B solution is cut into particles with uniform particle size distribution by a high-speed shear homogenizer. Preferably, the particle size distribution of the above microgel particles is 1-1000 $\mu$m.

In some specific embodiments of the invention, the drying method is vacuum freeze-drying or oven-drying. Preferred freeze-drying conditions are -20°C for 6 hours followed by -60°C for 48 hours; drying conditions are 30°C for 12 hours. In some specific embodiments of the invention, the sieving is the process of separating coarse and fine powders by means of a mesh-like tool (e.g., a vibrating sifter) after pulverizing the powders of widely differing coarseness and fineness. Preferably, the particle size distribution of said sifted powder is 20 mesh to 500 mesh.

In some embodiments of the invention, the polysaccharide-based biomaterial, a polysaccharide-based hydrogel material containing a pore structure is obtained by mixing the component A solution, the component B solution, and the pore-making agent provided in the third objective of the invention, and a polysaccharide-based sponge material is obtained by further drying.

Wherein, the component A is a polysaccharide-based polymer crosslinker modified by o-phthalaldehyde groups, and the component B is a water-soluble small molecule, a water-soluble synthetic macromolecule, a water-soluble natural macromolecule (e.g., proteins, nucleic acids, and polysaccharides) containing one or more of primary amine, biphenylamine, hydrazide, hydroxylamine or sulfhydryl group, and the number of primary amine, biphenylamine, hydrazide, hydroxylamine, or sulfhydryl group functionalities on a single molecule is not less than 2.

In some embodiments of the invention, the pore-making agent is an additive that causes a pore structure to be created in the material, including substances that readily decompose into gases, polymer microspheres, polyethylene glycol, polyvinylpyrrolidone, surfactants, water, and analogous components.

Wherein, substances that are easily decomposed into gases, such as ammonium bicarbonate, are added to the material and heat will release carbon dioxide and ammonia, which will overflow from the material to produce the pore structure. Polymer microspheres such as polymethyl methacrylate microspheres, silica microspheres and polystyrene microspheres.

Preferably, the pore-making agent is selected to be water, and the hydrogel is freeze-dried to a porous honeycomb sponge structure.

Preferably, the freeze-drying conditions are -20°C for 6 hours followed by -60°C for 48 hours.

The sixth objective of the invention is to provide applications of the polysaccharide-based dual-component hydrogel based on the fourth objective of the invention, selected from one of the following applications:

The application of the polysaccharide-based dual-component hydrogel in the preparation of a tissue repair product;

The application of the polysaccharide-based dual-component hydrogel in the preparation of tissue fluid leakage sealing products;

The application of the polysaccharide-based dual-component hydrogel in the preparation of tissue air leakage sealing products;

The application of the polysaccharide-based dual-component hydrogel in the preparation of hemostatic products.

Wherein, the tissue repair includes skin repair, abdominal cavity wall repair; the tissue fluid leakage blockage includes pancreatic fluid leakage blockage, cerebrospinal fluid leakage blockage, intestinal leakage blockage, gastric leakage blockage, etc.. The tissue air leakage blockage includes lung parenchyma leakage blockage, etc.. The hemostasis includes liver hemostasis, kidney hemostasis, spleen hemostasis, pancreatic hemostasis, bone fraction hemostasis, arterial hemostasis, and cardiac hemostasis, etc..

The seventh objective of the invention is to provide applications of polysaccharide-based biomaterials based on the fifth objective of the invention.

When the polysaccharide-based biomaterial is a polysaccharide-based membrane material, the application of the polysaccharide-based membrane material is selected from one of the following applications:

The application of the polysaccharide-based membrane material in the preparation of surgical wound closure products that do not require sutures;

The application of the polysaccharide-based membrane material in the preparation of patch products;

Wherein, patch products prepared from polysaccharide-based membrane materials can be used for abdominal hernia repair, etc..

When the polysaccharide-based biomaterial is a polysaccharide-based powder material, the application of the polysaccharide-based powder material is selected from one of the following applications:

The application of the polysaccharide-based powder material in the preparation of hemostatic products;

The application of the polysaccharide-based powder material in the preparation of cell, factor, and drug carrier products;

Wherein, hemostatic products prepared from the polysaccharide-based powder material can be used for hemostasis in minimally invasive surgery, liver hemostasis, kidney hemostasis, spleen hemostasis, pancreatic hemostasis, bone fraction hemostasis, arterial hemostasis, and cardiac hemostasis, etc.; cells, factors, and drug carriers include stem cell carriers, growth factor carriers, antimicrobial drug carriers, and anti-inflammatory drug carriers, etc..

When the polysaccharide-based biomaterial is a polysaccharide-based sponge material, the application of the polysaccharide-based sponge material is selected from one of the following applications:

The application of the polysaccharide-based sponge material in the preparation of wound dressing products;

The application of the polysaccharide-based sponge material in the preparation of hemostatic products.

When the polysaccharide-based sponge material is used to prepare hemostatic products, it can be applied to hemostasis in the liver hemostasis, kidney hemostasis, spleen hemostasis, pancreatic hemostasis, bone fraction hemostasis, arterial hemostasis, and cardiac hemostasis, and non-compressive penetrating injuries, etc..

Polysaccharides are a class of naturally occurring water-soluble polymers with a wide range of sources and excellent biocompatibility.

The invention provides a class of polysaccharide-based polymer crosslinkers modified with o-phthalaldehyde groups. The polysaccharide-based polymer crosslinkers modified with o-phthalaldehyde groups in the invention do not rely on artificially synthesized hydrophilic polymer backbones or complex chemical synthesis steps. The polysaccharide-based polymer crosslinkers provided by the invention have the advantages of a wide range of raw material sources and simple preparation methods.

The present invention also combines polysaccharide-based polymer crosslinkers with small molecules or polymer derivatives containing one or more of the following groups: primary amine, hydrazine, hydrazide, hydroxylamine, or sulfhydryl, to form a polysaccharide-based dual-component hydrogel material in an aqueous medium. This hydrogel material is crosslinked by two components: one is a polysaccharide-based polymer crosslinkers modified with o-phthalaldehyde groups, and the other is water-soluble small molecules, water-soluble synthetic polymers, or water-soluble natural polymers (such as proteins, nucleic acids, and polysaccharides), etc.. Each of the two components contains at least two of the corresponding functional groups. The polysaccharide-based dual-component hydrogel material provided by this invention has the advantages of being injectable, low swelling, and strong tissue adhesion, and can be used as a tissue adhesive, sealant, hemostatic agent, tissue engineering material, etc..

The invention also provides various forms of polysaccharide-based biomaterials (such as membranes, powders, and sponges) based on polysaccharide-based polymer crosslinkers or hydrogel materials. The dual-component hydrogels, membranes, powders, and sponges provided by the invention can be used as tissue adhesives, sealants, hemostats, tissue engineering materials, etc., and have broad biomedical application prospects. They are of great significance for hemostasis, tissue adhesion, and sealing in clinical practice.

## BRIEF DESCRIPTION OF DRAWINGS

Figure 1 shows the nuclear magnetic resonance hydrogen spectrum of the hyaluronic acid-based crosslinker (i.e., component A-1.2) in Embodiment 2.

Figure 2 shows the nuclear magnetic resonance hydrogen spectrum of the carboxymethyl cellulose-based crosslinker (i.e., component A-2.2) in Embodiment 3.

Figure 3 shows the precursor solution and photograph of the gel after gelation of the polysaccharide-based dual-component hydrogel (Formulation 7) in Embodiment 11.

Figure 4 shows a photograph of the polysaccharide-based dual-component hydrogel (Group A) and suture (Group B) applied to skin tissue repair at 7 days in Embodiment 16.

Figure 5 shows a photograph of the polysaccharide-based dual-component hydrogel (Group A) applied to pancreatic stump sealing for pancreatic juice leakage at 7 days post-surgery in Embodiment 18.

Figure 6 shows photographs of polysaccharide-based dual-component hydrogel (Group A) and bone wax (Group B) applied to cerebrospinal fluid leakage sealing in Embodiment 19.

Figure 7 shows comparison photographs of H&E staining results 10 days post-surgery for polysaccharide-based membrane material applied to surgical wound closure without suturing (Group A) and suturing group (Group B) in Embodiment 24.

Figure 8 shows the microscopic morphology of polysaccharide-based powder materials with different particle sizes under a microscope in Embodiment 26.

Figure 9 shows the statistical graph of colon length in mice after treatment with normal mice, polysaccharide-based powder materials loaded with budesonide (Group A), budesonide enema solution (Group B), and a blank group (Group C) for acute enteritis in Embodiment 29.

Figure 10 shows comparison photos of sponge materials (Group A) and commercially available absorbable hemostatic gauze (Group B) applied to spleen hemostasis in Embodiment 32.

Figure 11 shows photos of sponge materials (Group A) applied to cardiac hemostasis in Embodiment 33.

## DESCRIPTION OF EMBODIMENTS

This invention will be described in detail in combination with drawings and specific embodiments.

Embodiment 1: Synthesis of aldehyde pre-protected o-phthalaldehyde precursor derivatives (i.e., Compounds 1-13)

Synthesis of Compounds 1-3 (s = 2, t = 7):

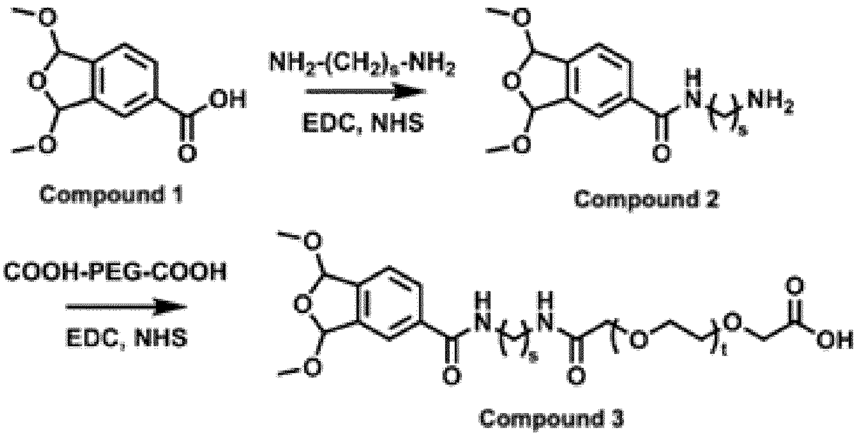


(1) Synthesis of Compound 1: The synthesis process refers to the literature Zhen Zhang, Chaoliang He, Yan Rong, Hui Ren, Tianran Wang, Zheng Zou, Xuesi Chen, National Science Review, Volume 8, Issue 4, April 2021, nwaa128. The method is publicly available. 1H NMR (400 MHz, DMSO-d6): $\delta$ = 12.74 (brs, 1H), 8.29 (d, J = 7.6 Hz, 1H), 8.12 (s, 1H), 7.65 (d, J = 7.6 Hz, 1H), 6.30 (s, 1H), 6.11 (s, 1H), 3.40-3.30 (m, 6H).

(2) Synthesis of Compound 2: Dissolve Compound 1 (10 g, 45 mmol) in 10 mL of anhydrous dichloromethane (DCM), and add 1-ethyl-3-(3-dimethylaminopropyl)carbodiimide (EDC, 10.38 g, 67 mmol) and N-hydroxy succinimide (NHS, 7.69 g, 67 mmol), stirring at room temperature for 30 minutes. Dissolve ethylenediamine (27.05 g, 0.45 mmol) in anhydrous DCM and stir. Quickly add the reaction mixture to the ethylenediamine solution and stir at room temperature for 12 hours. After the reaction is complete, remove most of the solvent. Extract the remaining compound three times with ethyl acetate, combine the organic phases, dry with anhydrous sodium sulfate, evaporate the solvent under reduced pressure, The crude product was purified by silica gel column chromatography to obtain Compound 2 (9.0 g, 75% yield). 1H NMR (400 MHz, DMSO-d6): $\delta$ = 8.18 (t, 1H), 8.07 (d, 1H), 7.80 (dd, 1H), 7.56 (dd, 1H), 6.05 (h, 2H), 4.59 (dt, 1H), 4.25 (dt, 1H), 3.38 (tt, J = 5.1 Hz, 2H), 3.34 (s, 3H), 3.03 (tt, J = 6.3, 2H).

(3) Synthesis of Compound 3: Dissolve $\alpha,\omega$-dicarboxylic acid polyethylene glycol (COOH-PEG-COOH, t = 7, 8.26 g) in 100 mL of anhydrous DCM, then add EDC (3.52 g, 22.7 mmol) and NHS (2.61 g, 22.7 mmol), and stir at room temperature for 30 minutes. Compound 2 (5 g, 18.8 mmol) was dissolved in anhydrous DCM (50 mL) and gradually added to the above system. The reaction was stirred at room temperature for 12 hours. After the reaction was complete, most of the solvent was removed. The remaining compound was extracted three times with ethyl acetate, the organic phases were combined, dried over anhydrous sodium sulfate, and the solvent was removed by rotary evaporation. The crude product was purified by silica gel column chromatography to give Compound 3 (10.5 g, 79% yield). 1H NMR (400 MHz, DMSO-d6): $\delta$ = 8.86 (s, 1H), 8.21 (d, J = 7.6 Hz, 1H), 7.93 (s, 1H), 7.61 (d, J = 7.6 Hz, 1H), 6.38 (s, 1H), 6.30 (s, 1H), 3.80-3.48 (m, 28H), 3.40-3.30 (m, 6H).

Synthesis of Compounds 4-8 (r = 2, s = 6, t = 7):

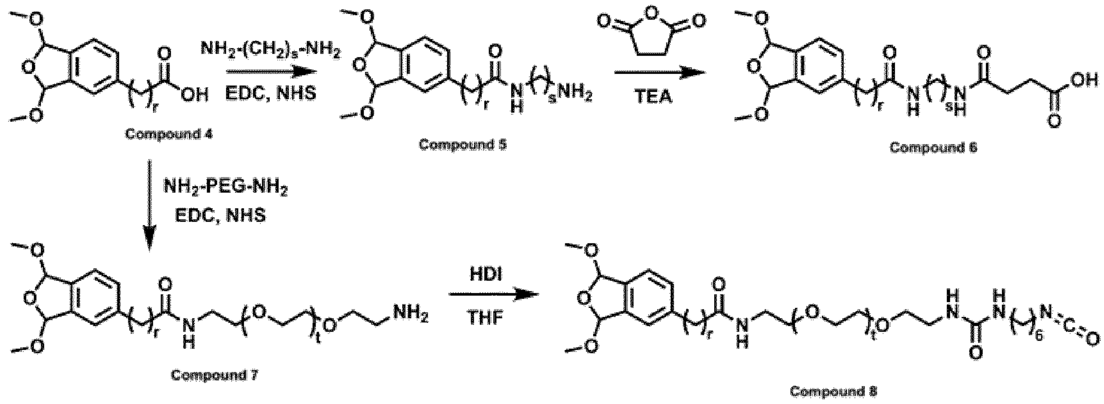

(1) Synthesis of Compound 4: The synthesis process refers to the literature Chun Ling Tung, Clarence T. T. Wong, Eva Yi Man Fung and Xuechen Li. Org. Lett. 2016, 18, 11, 2600-2603. The method is publicly available. 1H NMR (400 MHz, DMSO-d6): δ = 11.82 (s, 1H), 7.56 (dq, 1H), 7.43 (dd, 1H), 7.16 (ddt, 1H), 6.05 (m, 1H), 5.97 (q, 1H), 3.53 (dt, 2H), 3.40-3.30 (m, 6H).

(2) Synthesis of Compound 5: The synthesis process refers to the synthesis of Compound 2. 1H NMR (400 MHz, DMSO-d6): δ = 7.56 (t, 1H), 7.48 (m, 2H), 7.14 (ddt, 1H), 6.06 (s, 1H), 5.97 (q, 1H), 3.40-3.30 (m, 6H), 3.07 (q, 2H), 2.90-2.72 (m, 4H), 2.38 (t, J = 8.2 Hz, 2H), 1.87 (t, J = 6.5 Hz, 2H), 1.59-1.43 (m, 4H), 1.46-1.30 (m, 4H).

(3) Synthesis of Compound 6: Dissolve Compound 5 (5 g, 14.3 mmol) in 100 mL of toluene, add succinic anhydride (1.5 g, 15 mmol) and triethylamine (TEA, 2.1 mL, 15 mmol), and stir at room temperature for 4 hours. After the reaction is complete, remove the solvent by rotary evaporation, and purify the crude product by silica gel column chromatography to obtain Compound 6 (5.2 g, yield 80%). 1H NMR (400 MHz, DMSO-d6): δ = 11.87 (s, 1H), 7.62 (t, 1H), 7.56 (t, 1H), 7.44-7.36 (m, 2H), 7.14 (ddt, 1H), 6.08-6.02 (m, 1H), 5.97 (q, 1H), 3.40-3.30 (m, 6H), 3.19-3.03 (m, 4H), 2.77 (tt, J = 8.7 Hz, 2H), 2.55 (dd, 2H), 2.48-2.34 (m, 4H), 1.56-1.43 (m, 4H), 1.40-1.27 (m, 4H).

(4) Synthesis of Compound 7: Dissolve α,ω-diaminopolyethylene glycol ($NH_2$-PEG-NH2, t = 7, 8.8 g) in 50 mL of anhydrous DCM. Dissolve Compound 4 (5 g, 19.8 mmol) in anhydrous DCM (50 mL), and add EDC (4.65 g, 30 mmol) and NHS (3.45 g, 30 mmol), stirring at room temperature for 30 minutes. Gradually add the reaction mixture to the NH2-PEG-NH2 solution, stirring at room temperature for 12 hours. After the reaction is complete, remove most of the solvent, extract the remaining compound three times with ethyl acetate, combine the organic phases, dry with anhydrous sodium sulfate, remove the solvent by rotary evaporation, and purify the crude product by silica gel column chromatography to obtain Compound 7 (10.4 g, 81% yield). 1H NMR (400 MHz, DMSO-d6): δ = 7.43-7.36 (m, 2H), 7.14 (ddt, 1H), 3.61-3.43 (m, 28H), 3.36-3.26 (m, 6H), 2.88-2.71 (m, 2H), 2.54-2.36 (m, 2H).

(5) Synthesis of Compound 8: Dissolve hexamethylene diisocyanate (HDI, 1.28 g, 7.6 mmol) in anhydrous tetrahydrofuran (THF, 50 mL). Compound 7 (5 g, 7.6 mmol) was dissolved in anhydrous THF (50 mL) and slowly added dropwise to the rapidly stirring HDI solution. The reaction was stirred at room temperature for 2 hours. After removing the solvent by rotary evaporation, the residue was redissolved in a small amount of DCM, poured into n-pentane to precipitate the solid, and the process was repeated twice. The solid was then dried under vacuum to obtain Compound 8 (5.5 g, 92% yield). 1H NMR (400 MHz, DMSO-d6): δ = 7.53 (t, 1H), 7.44-7.36 (m, 2H), 7.14 (ddt, 1H), 6.047 (s, 1H), 5.97 (s, 1H), 5.87 (t, 1H), 5.65 (t, 1H), 3.63-3.51 (m, 32H), 3.36-3.28 (m, 4H), 3.39-3.32 (m, 6H), 3.31-3.18 (m, 4H), 3.10 (tdd, J = 5.7, 4.7, 1.0 Hz, 2H), 2.90-2.71 (m, 4H), 2.41 (t, J = 8.3 Hz, 2H), 1.87 (t, J = 6.5 Hz, 2H), 1.59-1.27 (m, 8H).

Synthesis of Compounds 9-13:

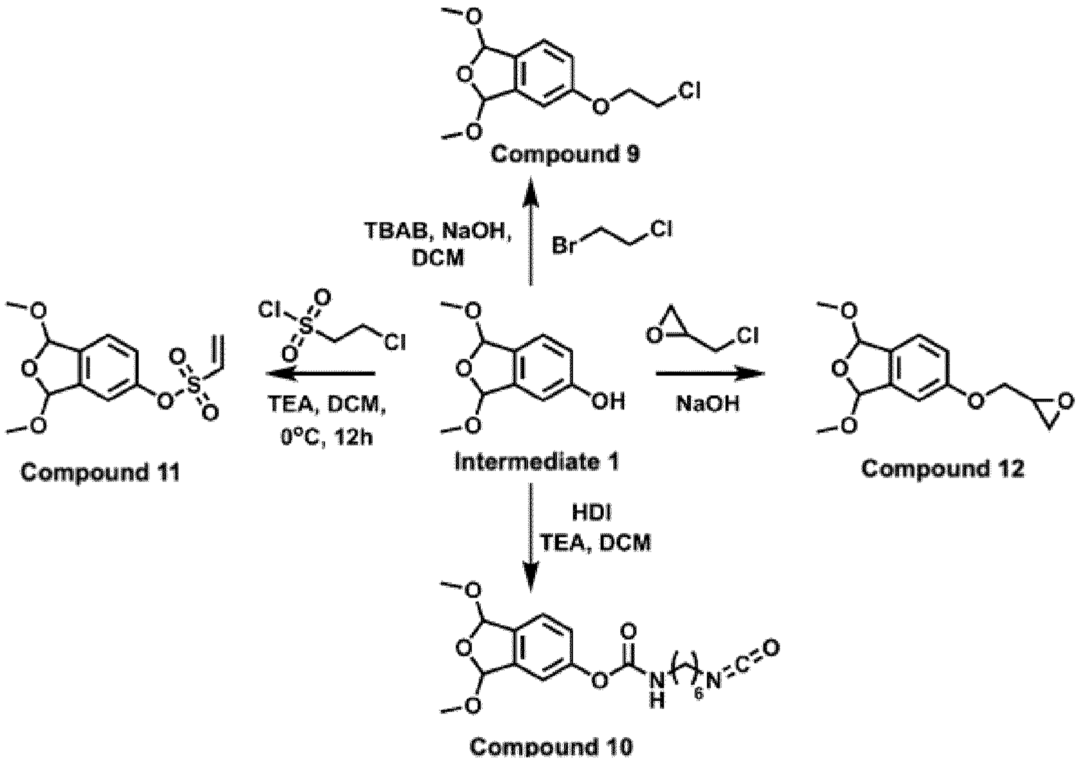


Compound 9

Compound 11

Intermediate 1

Compound 12

Compound 10

(1) Synthesis of Compound 9: Dissolve intermediate 1 (5 g, 25.5 mmol) in anhydrous DCM (40 mL), add 0.2 M NaOH solution (85 mL), then add tetrabutylammonium bromide (TBAB, 0.82 g, 2.55 mmol), and stir at room temperature for 30 min. Add 1-bromo-2-chloroethane (8.41 mL, 0.102 mol) dropwise, and stir the reaction at room temperature for 48 hours. Then separate the phases, the organic phase was washed successively with brine, dried over anhydrous sodium sulfate, filtered, and concentrated under reduced pressure. The crude product was purified on a silica gel column to obtain Compound 9 (5.6 g, 85% yield). 1H NMR (400 MHz, DMSO-d6): $\delta$ = 7.45 (dd, 1H), 7.15 (dd, 1H), 6.88 (dd, 1H), 6.07-5.97 (m, 2H), 4.24 (q, J = 2.2 Hz, 2H), 3.81 (t, J = 2.2 Hz, 2H), 3.37-3.34 (s, 6H).

(2) Synthesis of Compound 10: Dissolve HDI (6.43 g, 38.3 mmol) in anhydrous DCM. Dissolve intermediate 1 (5 g, 25.5 mmol) in anhydrous DCM and add TEA (3.53 mL, 25.5 mmol). Slowly add the above mixture to the rapidly stirring HDI solution, and stir at room temperature for 30 minutes. After removing the solvent by rotary evaporation, redissolve in a small amount of DCM, pour into n-pentane to precipitate the solid, repeat twice, and obtain Compound 10 (9.76 g, yield 70%). 1H NMR (400 MHz, DMSO-d6): $\delta$ = 7.56 (dd, J = 8.4, 1H), 7.40 (s, 1H), 7.07 (dd, J = 8.3, 1H), 6.34 (t, J = 5.3 Hz, 1H), 6.03 - 5.93 (m, 2H), 3.38-3.31 (s, 6H), 3.20-3.13 (m, 2H), 3.06 (q, J = 5.5 Hz, 2H), 1.67-1.27 (m, 8H).

(3) Synthesis of Compound 11: Under anhydrous and oxygen-free conditions, dissolve intermediate 1 (5 g, 25.5 mmol) in anhydrous DCM. Under an ice bath at 0°C, slowly add 2-chloroethane sulfonyl chloride (5 g, 30.6 mmol) to the solution. After reacting for 10 minutes, slowly add anhydrous TEA (4.23 mL, 30.6 mmol) and react at room temperature for 12 hours. After the reaction is complete, remove the solvent by rotary evaporation, and purify the crude product using silica gel chromatography to obtain Compound 11 (6.9 g, 95% yield). 1H NMR (400 MHz, DMSO-d6): $\delta$ = 7.48-7.41 (m, 1H), 7.35 (s, 1H), 7.08 (dd, J = 8.2, 1H), 6.26 (dd, J = 18.5, 1H), 6.15 (dd, J = 18.4, 1H), 6.03 (dq, J = 2.9, 1.7 Hz, 2H), 3.41-3.33 (s, 6H).

(4) Synthesis of Compound 12: Suspended intermediate 1 (5 g, 25.5 mmol) in 30 mL of water, added 1.5 M NaOH solution (10 mL), and the system gradually became clear. Stirred for 40 minutes, added epichlorohydrin (7.1 g, 76.5 mmol), and stirred at room temperature for 24 hours. After the reaction was complete, most of the solvent was removed, the remaining compound was extracted three times with ethyl acetate, the organic phases were combined, dried over anhydrous sodium sulfate, and the solvent was removed by rotary evaporation. The crude product was purified on a silica gel column to obtain Compound 12 (5.8 g, 90% yield). 1H NMR (400 MHz, DMSO-d6): $\delta$ = 7.45 (s, 1H), 7.14 (s, 1H), 6.91 (dd, J = 8.3, 1H), 6.07-5.99 (m, 2H), 4.11 (d, J = 3.3 Hz, 2H), 3.42 (s, 1H), 3.38-3.32 (m, 6H), 2.91-2.78 (m, 2H).

Embodiment 2: Synthesis of representative Components A-1.1 to A-1.4 of o-phthalaldehyde-modified hyaluronic acid-based crosslinkers

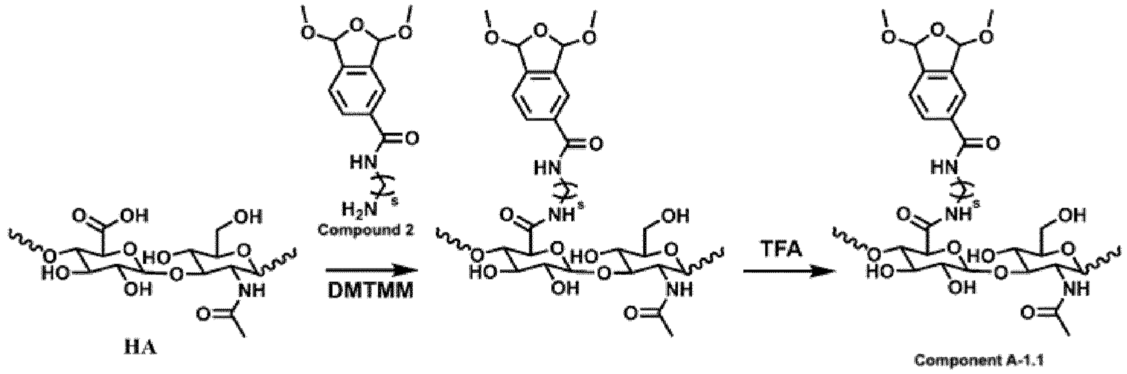


HA

Component A-1.1

(1) Synthesis of Component A-1.1: Dissolve hyaluronic acid (HA, 5 g, 890 kDa) in 500 mL of 0.01 mol/L 2-(N-morpholine) ethane sulfonic acid (MES) buffer solution (pH = 5.2), stir until completely dissolved, dissolve Compound 2 (0.54 g, 2 mmol, s = 2) in 10 mL of dimethyl sulfoxide (DMSO) and add it to the reaction mixture. Add 4-(4,6-dimethoxy-2-triazine)-4-methylmorpholine hydrochloride (DMTMM, 1.4 g, 5 mmol) in three portions to the reaction system, each dissolved in 3 mL of MES buffer solution, with an interval of 1 h, and reacted at 37 °C for 24 hours. After the reaction, the pH was adjusted to 8-9, stirred for 1 hour, dialyzed with deionized water for 2-3 days (MWCO 14,000), collected the product, added 10% trifluoroacetic acid, reacted for 1 hour, adjusted to neutral, and then dialyzed the reaction system with deionized water for 1 day (MWCO 14,000), Collect the product and freeze-dry it to obtain Component A-1.1. The labeling of Compound 2 on HA was identified by nuclear magnetic hydrogen spectrum (1H NMR). Collect the supernatant of the reaction system and determine the amount of residual Compound 2 in the supernatant by high-performance liquid chromatography (HPLC). The labeling rate of Compound 2 was calculated to be approximately 6.78% *(w/w).*

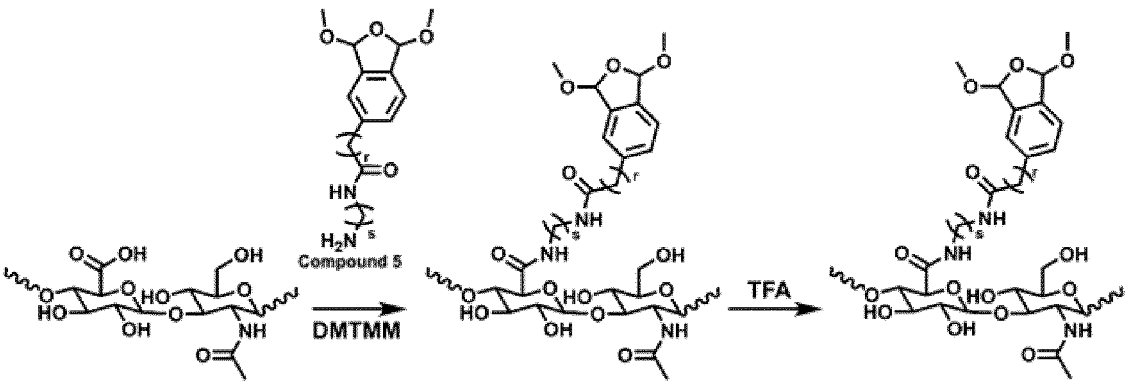


HA

Component A-1.2

(2) Synthesis of Component A-1.2: Dissolve HA (5 g, 340 kDa) in 250 mL MES buffer solution (0.01 M, pH). Dissolve compound 5 (420 mg, 1.2 mmol, r = 2, s = 6) in 10 mL DMSO and add to the reaction system. During dialysis, use a dialysis bag with a MWCO of 7000. The remainder of the synthesis process follows the synthesis of Component A-1.1. The representative hyaluronic acid-based crosslinking agent (i.e., Component A-1.2) in Embodiment 2 is shown in Figure 1. The labeling of Compound 5 on HA was identified using the 1H NMR. The supernatant of the reaction system was collected, and the residual amount of Compound 5 in the supernatant was determined by HPLC. The labeling rate of Compound 5 was calculated to be approximately 4.21% *(w/w).*

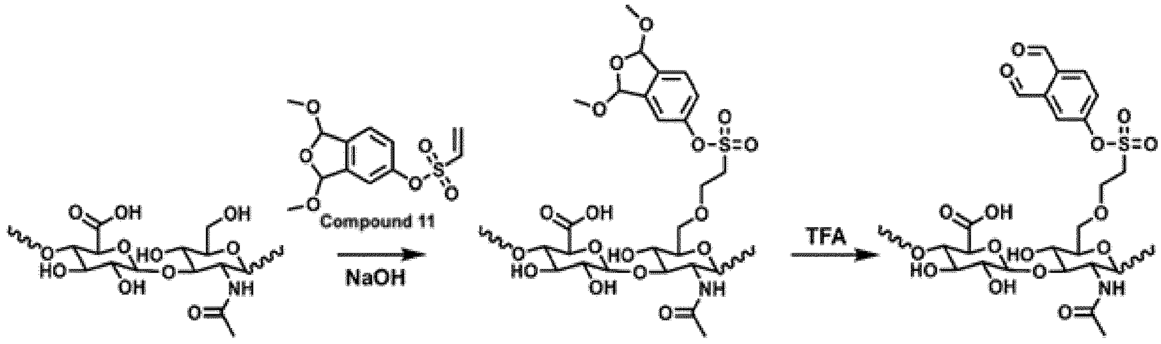


HA

Component A-1.3

(3) Synthesis of Component A-1.3: Dissolve HA (5 g, 1.2 MDa) in 50 mL of 0.2 M NaOH solution and stir until completely dissolved. Add Compound 11 (343 mg, 1.2 mmol) to the above system and react at 50°C for 6 hours. After the reaction, neutralize the pH to 7 with dilute hydrochloric acid, and dialysis with deionized water for 2-3 days (MWCO 14000). Collect the product, add 10% trifluoroacetic acid, react for 1 hour, adjust to neutral pH, then dialysis the reaction system with deionized water for 1 day (MWCO 14,000), collect the product, freeze-dry, and obtain Component A-1.3. The labeling of compound 11 on HA was identified by 1H NMR. Collect the supernatant from the reaction system, determine the amount of residual Compound 11 in the supernatant by HPLC, and calculate the labeling rate of Compound 11 to be approximately 4.12% *(w/w).*

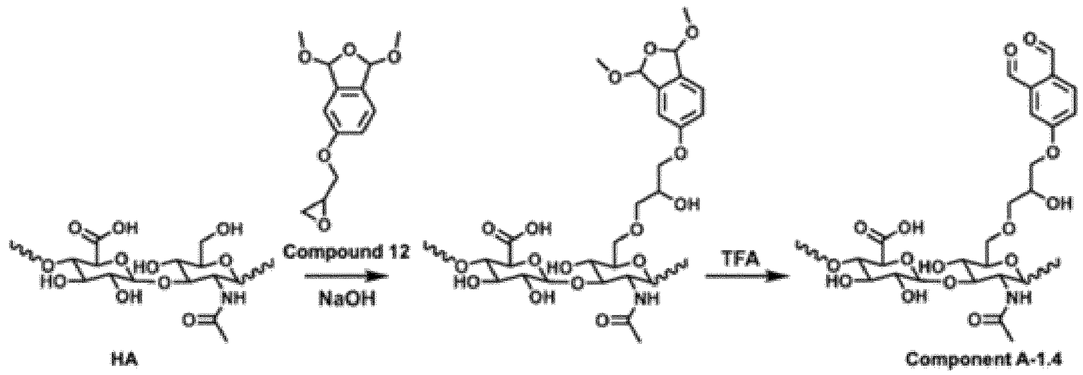


HA

Component A-1.4

(4) Synthesis of Component A-1.4: Dissolve HA (5 g, 1.2 MDa) in 50 mL of 0.25 M NaOH solution and stir until completely dissolved. Add Compound 12 (302 mg, 1.2 mmol) to the above system and react at 40°C for 12 hours. After the reaction, neutralize the pH to 7 with dilute hydrochloric acid, and dialysis with deionized water for 2-3 days (MWCO 14000). Collect the product, add 10% trifluoroacetic acid, react for 1 hour, adjust to neutral pH, then dialysis the reaction system with deionized water for 1 day (MWCO 14,000), collect the product, freeze-dry, and obtain Component A-1.4. The labeling of Compound 12 on HA was identified by 1H NMR. Collect the supernatant from the reaction system and determine the amount of residual Compound 12 in the supernatant by HPLC. The labeling rate of Compound 12 is calculated to be approximately 4.01% *(w/w).*

The above structural formulae and embodiments provide examples of the structure and synthesis methods of o-phthalaldehyde-modified hyaluronic acid-based polymer crosslinkers. Those skilled in the art will be able to conceive of other o-phthalaldehyde-protected derivatives that can be used to synthesize the o-phthalaldehyde-modified hyaluronic acid-based polymer crosslinkers described in this application.

Embodiment 3: Synthesis of representative Components A-2.1 to A-2.4 of o-phthalaldehyde-modified carboxymethyl cellulose-based crosslinkers

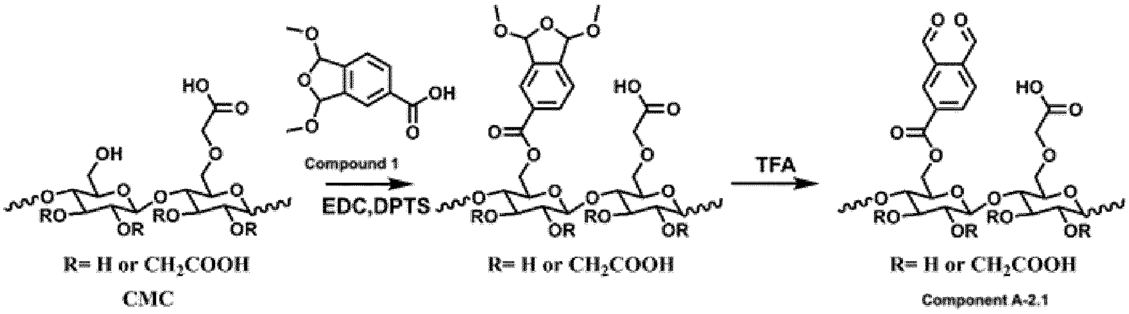


(1) Synthesis of Component A-2.1: Dissolve carboxymethyl cellulose (CMC, 5 g, viscosity (2%, 25°C) 1200 mPa·s, carboxymethyl substitution degree 0.7) in 500 mL of deionized water and stir until completely dissolved. Add Compound 1 (269 mg, 1.2 mmol), ethyl-3-(3-dimethylaminopropyl)carbodiimide (EDC, 1.86 g, 12 mmol), and dimethylaminopyridine p-toluenesulfonate (DPTS, 3 g, 12 mmol) to the solution in sequence. React at room temperature for 24 hours. After the reaction, dialysis with a sodium chloride aqueous solution containing dilute hydrochloric acid (pH = 4) for 2-3 days (MWCO 14,000), collect the product, add 10% trifluoroacetic acid, react for 1 hour, adjust to neutrality, then dialysis the reaction system with deionized water for 1 day (MWCO 14,000), collect the product, and freeze-dry. yielding Component A-2.1. The labeling of Compound 1 on CMC was identified by 1H NMR. The supernatant of the reaction system was collected, and the residual amount of Compound 1 in the supernatant was determined by HPLC, yielding a labeling rate of approximately 2.48% *(w/w).*

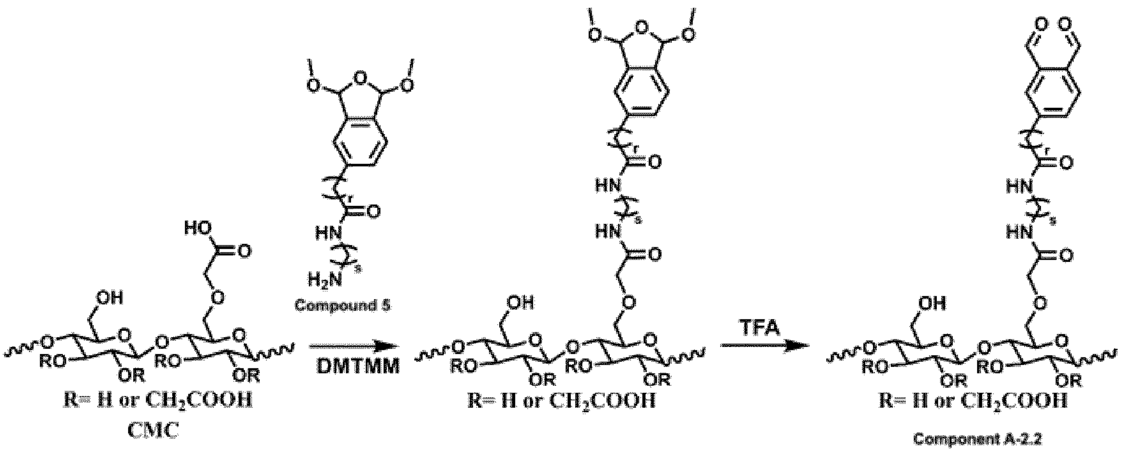


(2) Synthesis of Component A-2.2: Dissolve CMC (5 g, viscosity (2%, 25°C) = 675 mPa·s, carboxymethyl substitution degree = 0.7) in 250 mL of MES buffer solution (0.01 M, pH = 5.2) and stir until completely dissolved. Dissolve Compound 5 (280 mg, 0.8 mmol, r = 2, s = 6) in DMSO and add it to the reaction system. The remaining synthesis process follows that of Component A-1.2. The nuclear magnetic resonance hydrogen spectrum of the representative carboxymethyl cellulose-based crosslinking agent (i.e., Component A-2.2) in Embodiment 3 is shown in Figure 2. The labeling of Compound 5 on CMC was identified by the 1H NMR. The supernatant of the reaction system was collected, and the amount of residual Compound 5 in the supernatant was determined by HPLC, yielding a labeling rate of approximately 3.31% *(w/w).*

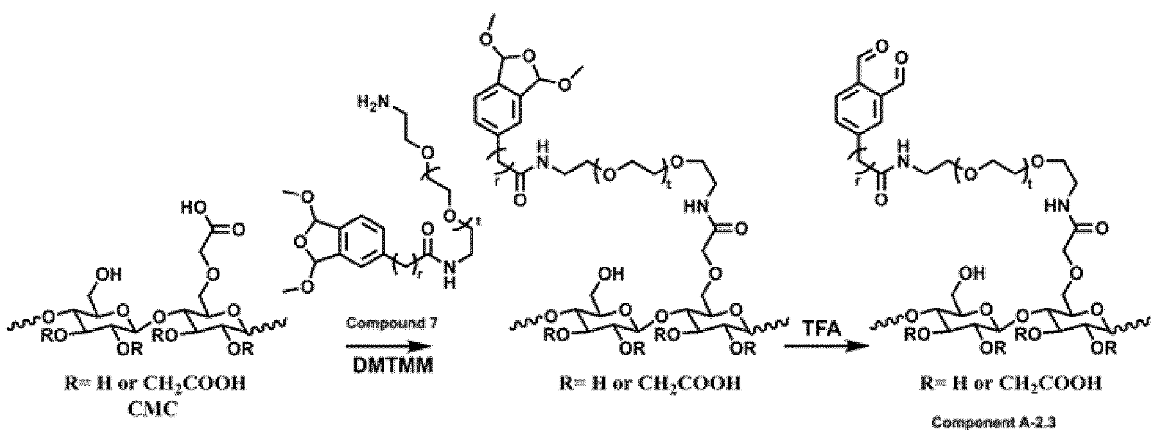


(3) Synthesis of Component A-2.3: Dissolve CMC (5 g, viscosity (2%, 25°C) = 675 mPa·s, carboxymethyl substitution degree = 0.7) in 250 mL of MES buffer solution (0.01 M, pH = 5.2) and stir until completely dissolved; weigh compound 5 (280 mg, 0.8 mmol, r = 2, s = 6) in DMSO and add it to the reaction system. The remaining synthesis process follows that of Component A-1.2. The nuclear magnetic resonance hydrogen spectrum of the representative carboxymethyl cellulose-based crosslinking agent (i.e., Component A-2.2) in Embodiment 3 is shown in Figure 2. The labeling of Compound 5 on CMC was identified by the 1H NMR. The supernatant of the reaction system was collected, and the amount of residual Compound 5 in the supernatant was determined by HPLC, yielding a labeling rate of approximately 3.31% *(w/w).*

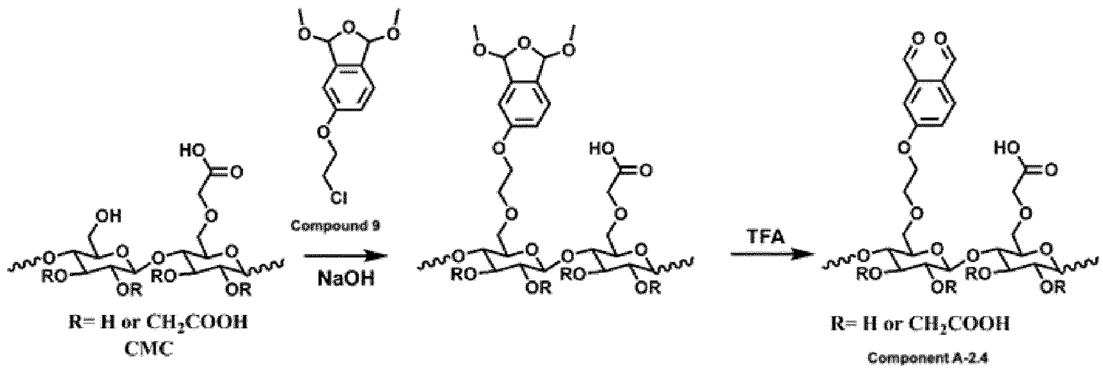


(4) Synthesis of Component A-2.4: Dissolve CMC (5 g, viscosity (2%, 25°C) = 2000 mPa·s, carboxymethyl substitution degree = 0.85) in 50 mL of 0.25 M NaOH solution and stir until completely dissolved. Add Compound 9 (309 mg, 1.2 mmol) to the above system and react at 40°C for 12 hours. After the reaction, neutralize the pH to 7 with dilute hydrochloric acid, dialysis with deionized water for 2-3 days (MWCO 14,000), collect the product, add 10% trifluoroacetic acid, react for 1 hour, then adjust to neutral pH. Then, the reaction mixture was dialyzed with deionized water for 1 day (MWCO 14,000), the product was collected and freeze-dried, yielding Component A-2.4. The labeling of Compound 9 on CMC was identified by 1H NMR. The supernatant of the reaction mixture was collected, and the residual amount of Compound 9 in the supernatant was determined by HPLC, yielding a labeling rate of approximately 3.01% *(w/w).*

The above structural formulae and embodiments provide examples of the structure and synthesis methods of o-phthalaldehyde-modified carboxymethyl cellulose-based polymer crosslinkers. Those skilled in the art will be able to conceive of other o-phthalaldehyde-protected derivatives that can be used to synthesize the o-phthalaldehyde-modified carboxymethyl cellulose-based polymer crosslinkers described in this application.

Embodiment 4: Synthesis of representative Components A-3.1 to A-3.3 of o-phthalaldehyde-modified alginate-based crosslinkers

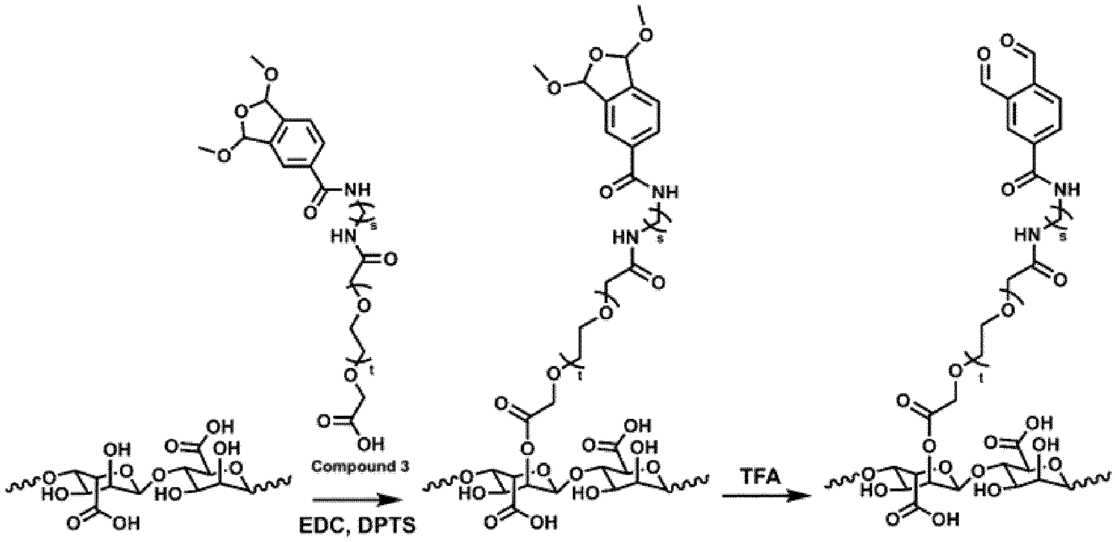


Alg

Component A-3.1

(1) Synthesis of Component A-3.1: Dissolve sodium alginate (Alg, 5 g, viscosity (2%, 25°C) = 50 mPa·s) in 250 mL of deionized water and stir until completely dissolved. Add Compound 3 (0.65 g, 1 mmol, s = 2, t = 7), EDC (1.6 g, 10 mmol), and DPTS (2.5 g, 10 mmol) sequentially to the above solution; during dialysis, a dialysis bag with a MWCO of 3500 is selected, and the remaining synthesis process follows the synthesis procedure of Component A-2.1. The labeling of Compound 3 on Alg is identified by 1H NMR. Collect the supernatant from the reaction system. Determine the amount of residual Compound 3 in the supernatant by HPLC. The labeling efficiency of Compound 3 is approximately 2.63% *(w/w).*

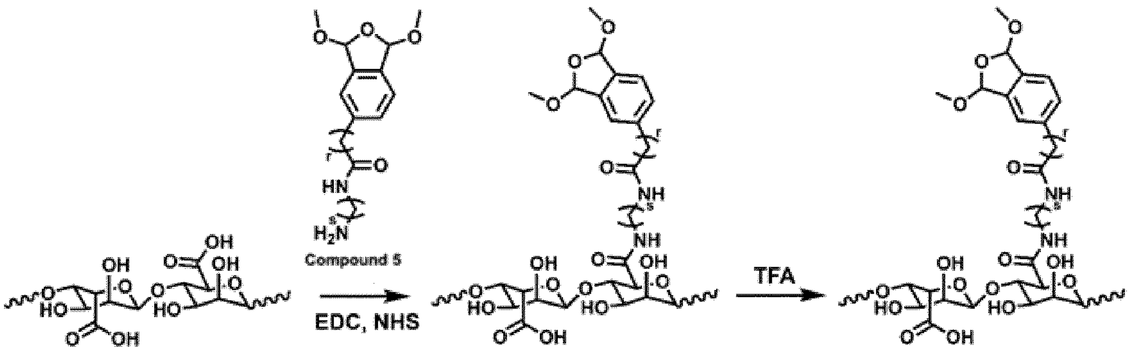


Alg

Component A-3.2

(2) Synthesis of Component A-3.2: Dissolve Alg (5 g, viscosity (2%, 25°C) = 980 mPa·s) in 250 mL of MES buffer solution (0.01 M, pH = 5.2) and stir until completely dissolved. Add EDC (1.54 g, 10 mmol) and NHS (1.14 g, 10 mmol) and stir at room temperature for 30 minutes. Add Compound 5 (350 mg, 1 mmol, r = 2, s = 6) to the reaction mixture. Stir at room temperature for 24 hours. After the reaction, neutralize the pH to 7 with dilute hydrochloric acid, dialysis with deionized water for 2-3 days (MWCO 7000), collect the product, add 10% trifluoroacetic acid, react for 1 hour, then adjust to neutral pH. Then, the reaction mixture was dialyzed with deionized water for 1 day (MWCO 7000), the product was collected and freeze-dried, yielding Component A-3.2. The labeling of Compound 5 on Alg was identified by 1H NMR. The supernatant of the reaction mixture was collected, and the residual amount of Compound 5 in the supernatant was determined by HPLC, yielding a labeling rate of approximately 2.57% *(w/w).*

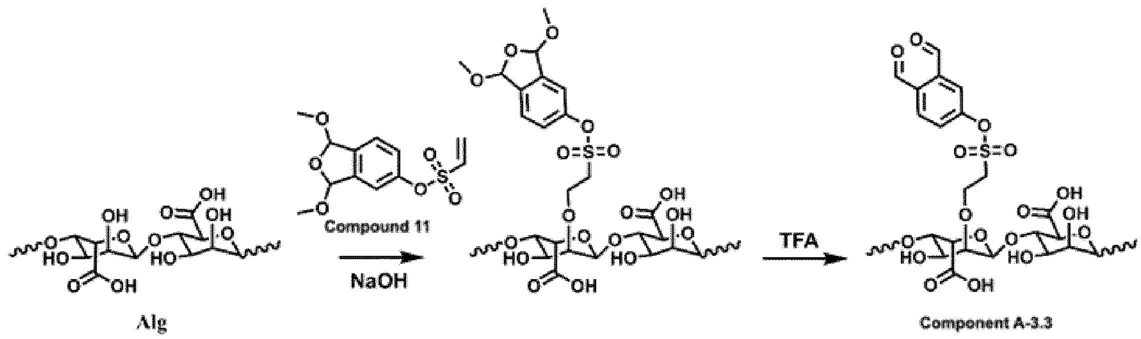


(3) Synthesis of Component A-3.3: Dissolve Alg (5 g, viscosity (2%, 25°C) = 980 mPa·s) in 40 mL of 0.2 M NaOH solution and stir until completely dissolved; add Compound 11 (286 mg, 1 mmol) to the above system, and the remaining synthesis process follows that of Component A-1.3. The labeling of Compound 11 on Alg was identified by 1H NMR. The supernatant of the reaction system was collected, and the amount of residual Compound 11 in the supernatant was determined by HPLC, yielding a labeling rate of approximately 2.20% *(w/w).*

The above structural formulae and embodiments provide examples of the structure and synthesis methods of o-phthalaldehyde-modified alginate-based polymer crosslinkers. Those skilled in the art will be able to conceive of other o-phthalaldehyde-protected derivatives that can be used to synthesize the o-phthalaldehyde-modified alginate-based polymer crosslinkers described in this application.

Embodiment 5: Synthesis of representative Components A-4.1 and A-4.2 of o-phthalaldehyde-modified carboxymethyl starch-based crosslinkers

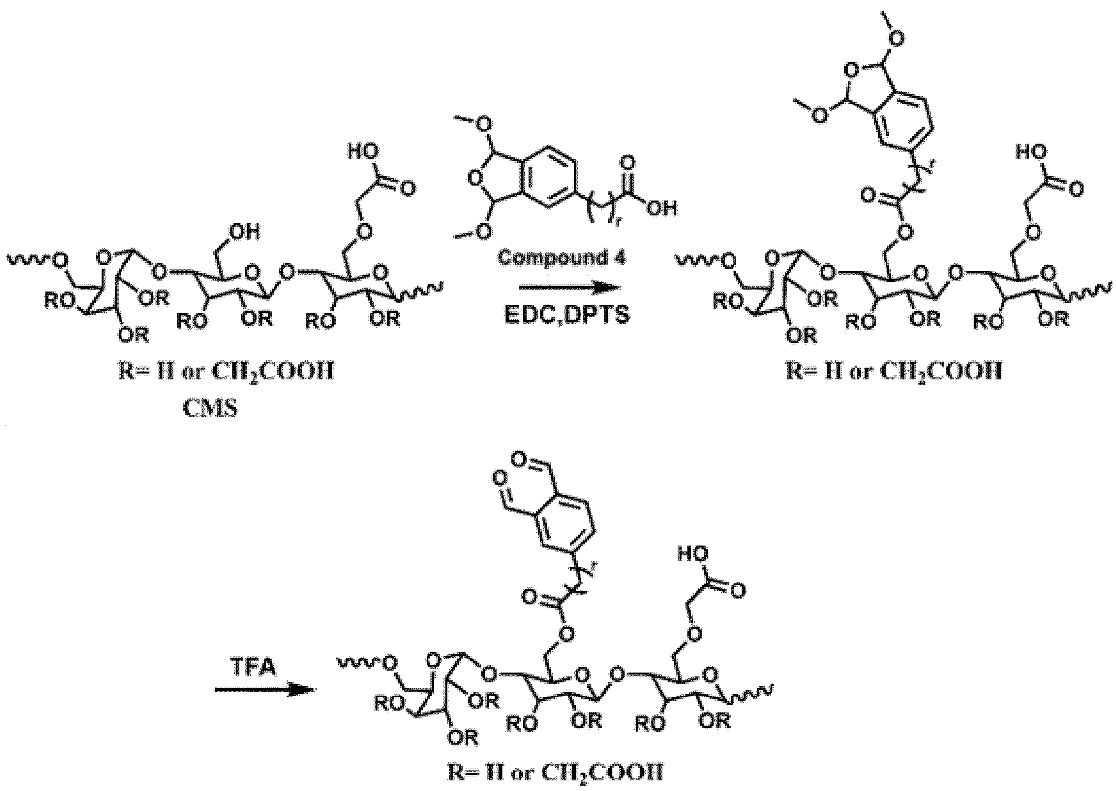


Component A-4.1

(1) Synthesis of Component A-4.1: Disperse sodium carboxymethyl starch (CMS, Type A, 5 g, pH 5.0-7.5) in 250 mL of deionized water. Add Compound 4 (0.3 g, 1.2 mmol, r = 2), EDC (1.86 g, 12 mmol), and DPTS (3 g, 12 mmol)

sequentially to the above system. React at room temperature for 24 hours. After the reaction, precipitate with 10 times the volume of anhydrous ethanol, collect the white powder, redisperse the solid in 250 mL of deionized water, add 10% trifluoroacetic acid, react for 1 h, and adjust the pH to neutral. The precipitate was redissolved in 10 volumes of anhydrous ethanol, yielding a white powder, which was identified as Component A-4.1. The labeling of Compound 4 on the CMS was confirmed by 1H NMR. The supernatant from the reaction system was collected, and the residual amount of Compound 4 in the supernatant was determined by HPLC, yielding a labeling efficiency of approximately 2.15% *(w/w).*

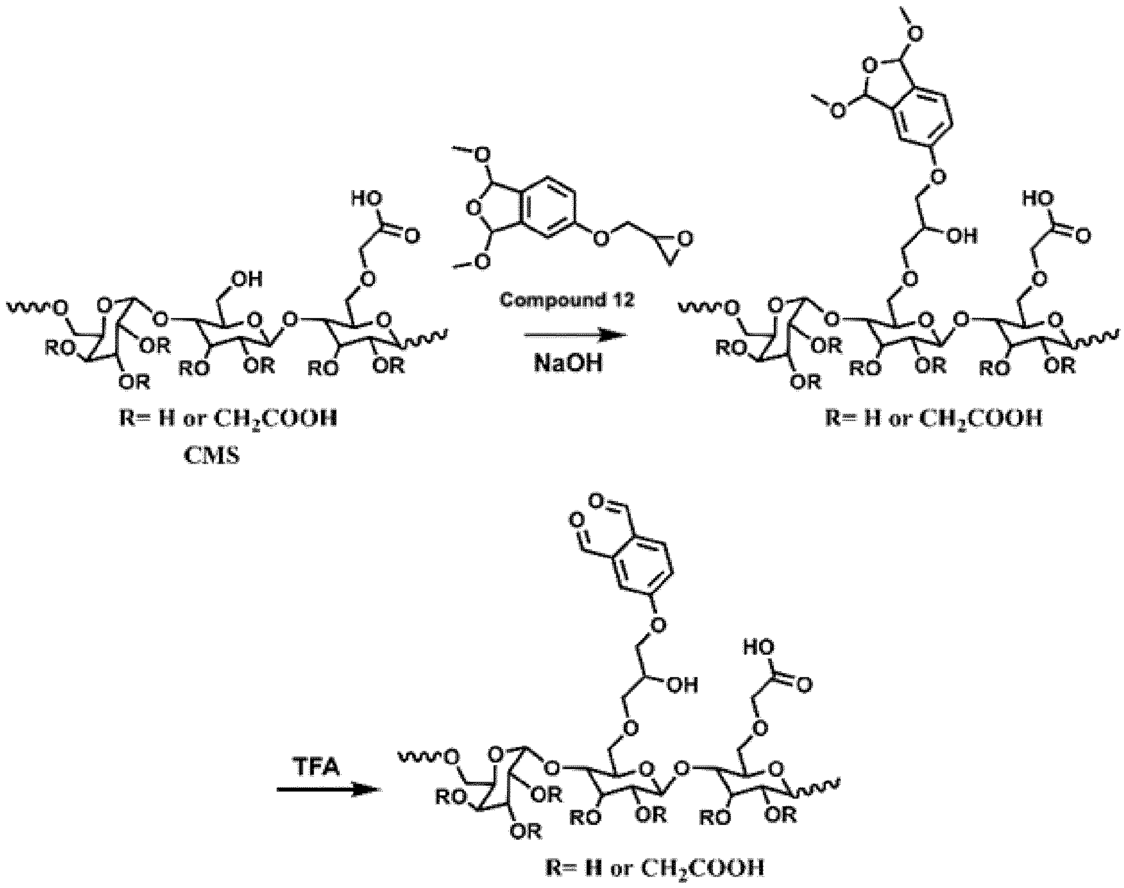


Component A-4.2

(2) Synthesis of Component A-4.2: Dissolve CMS (Type A, 5 g, pH 5.0-7.5) in 40 mL of 0.25 M NaOH solution. Add Compound 12 (0.3 g, 1.2 mmol) to the above system; finally, precipitate with 10 times the volume of anhydrous ethanol to obtain a white powder. The remaining synthesis process follows that of Component A-1.4. The labeling of Compound 12 on CMS was identified by 1H NMR. The supernatant of the reaction system was collected, and the residual amount of Compound 12 in the supernatant was determined by HPLC, yielding a labeling rate of approximately 1.78% *(w/w).*

The above structural formulae and embodiments provide examples of the structure and synthesis methods of o-phthalaldehyde-modified carboxymethyl starch-based polymer crosslinkers. Those skilled in the art will be able to conceive of other o-phthalaldehyde-protected derivatives that can be used to synthesize the o-phthalaldehyde-modified carboxymethyl starch-based polymer crosslinkers described in this application.

Embodiment 6: Synthesis of representative Components A-5.1 and A-5.2 of o-phthalaldehyde-modified hydroxyethyl cellulose-based crosslinkers

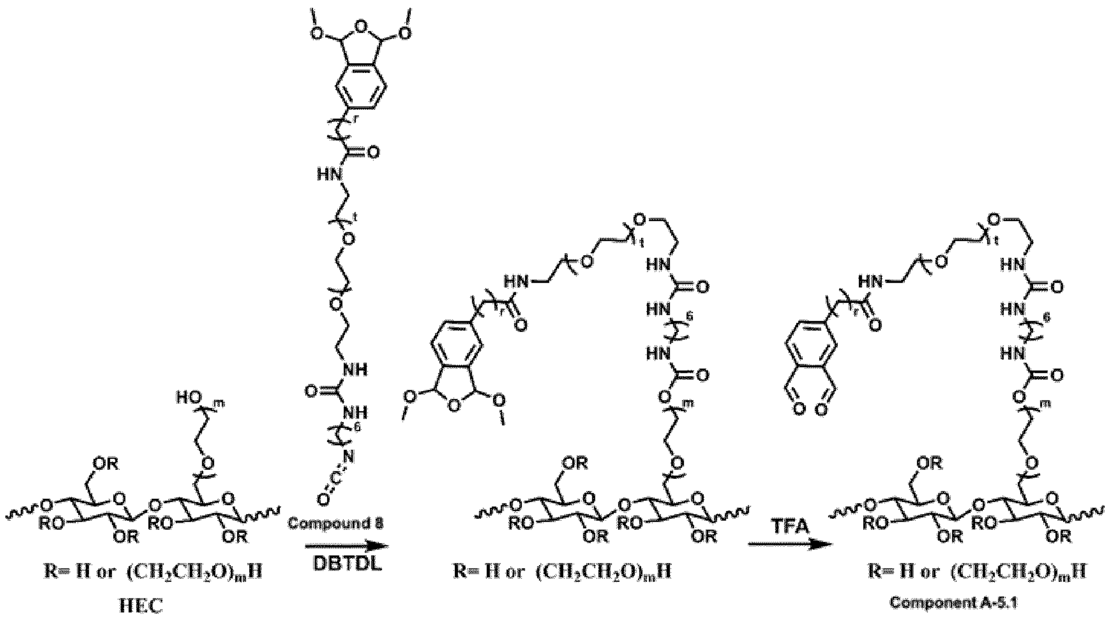


(1) Synthesis of Component A-5.1: Dissolve hydroxyethyl cellulose (HEC, Mw = 1 MDa, 5 g) in 250 mL of anhydrous DMSO and stir until completely dissolved. Dissolve Compound 8 (1.63 g, 2 mmol, r = 2, t = 7) in 5 mL of DMSO and add it to the above system. and add dibutyltin dilaurate (13 μL, 20 μmol) as a catalyst. Stir the reaction at 70°C for 24 hours. After the reaction, slowly add an equal volume of ice water, dialysis with deionized water for 2-3 days (MWCO 14,000), collect the product, add 10% trifluoroacetic acid, react for 1 hour, then adjust to neutral pH, followed by dialysis of the reaction system with deionized water for 1 day (MWCO 14,000). Collect the product and freeze-dry to obtain Component A-5.1. The labeling of Compound 8 on HEC was identified by 1H NMR. Collect the supernatant from the reaction system. Determine the amount of residual Compound 8 in the supernatant by HPLC, and calculate the labeling efficiency of Compound 8 to be approximately 4.12% *(w/w)*.

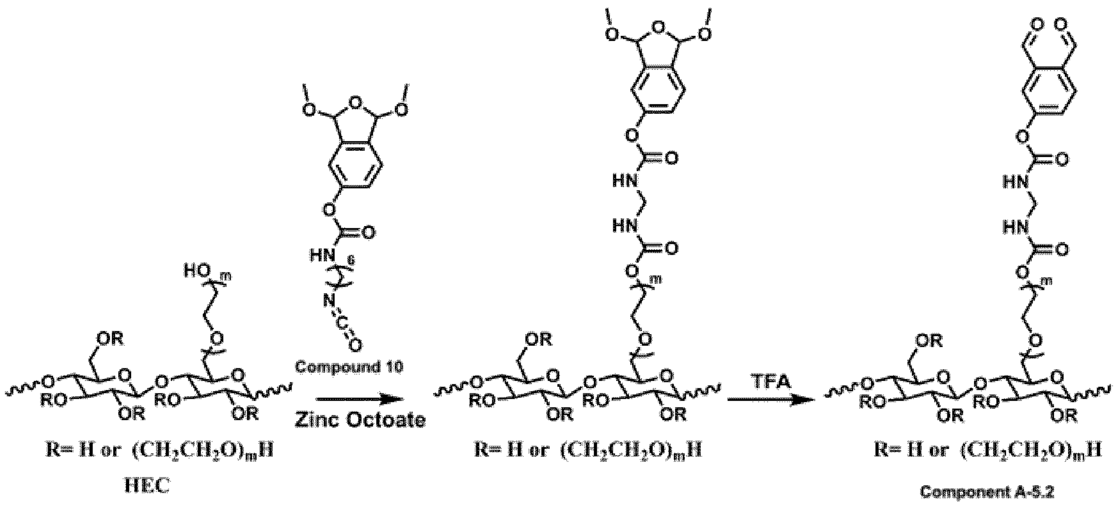


(2) Synthesis of Component A-5.2: Dissolve HEC (Mw = 300 kDa, 5 g) in 250 mL of anhydrous DMSO and stir until completely dissolved. Dissolve Compound 10 (0.73 g, 2 mmol) in 5 mL of DMSO and add it to the above system. and add zinc octoate (6 μL, 20 μmol) as a catalyst. Stir the reaction mixture at 70°C for 24 h. After the reaction, slowly add an equal volume of ice water, dialysis with deionized water for 2-3 days (MWCO 14,000), collect the product, add 10% trifluoroacetic acid, react for 1 h, then adjust to neutrality, then dialysed the reaction mixture with deionized water for 1 day (MWCO 7000), collected the product, and freeze-dried it to obtain Component A-5.2. The labeling of Compound 10 on HEC was identified by 1H NMR. The supernatant of the reaction mixture was collected, and the amount of residual compound 10 in the supernatant was determined by HPLC, yielding a labeling rate of approximately 3.14% *(w/w)*.

The above structural formulae and embodiments provide examples of the structure and synthesis methods of o-phthalaldehyde-modified hydroxyethyl cellulose-based polymer crosslinkers. Those skilled in the art will be able to conceive of other o-phthalaldehyde-protected derivatives that can be used to synthesize the o-phthalaldehyde-modified hydroxyethyl cellulose-based polymer crosslinkers described in this application.

Embodiment 7: Synthesis of representative Components A-6.1 of o-phthalaldehyde-modified heparin-based crosslinkers

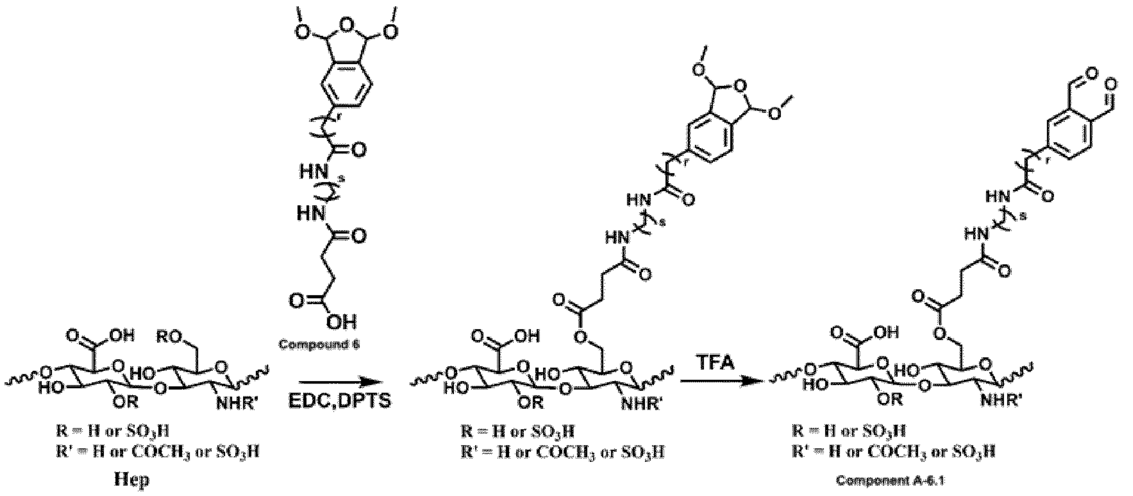


Dissolve heparin sodium (Hep, 5 g, 15 kDa) in 150 mL of deionized water and stir until completely dissolved. Add Compound 6 (0.97 g, 1.2 mmol, r = 2, t = 6), EDC (1.86 g, 12 mmol), and DPTS (3 g, 12 mmol) to the solution in sequence. React at room temperature for 24 hours. After the reaction, dialysis with a sodium chloride solution containing dilute hydrochloric acid (pH = 4) is performed for 2-3 days (MWCO 14,000). The product is collected, and 10% trifluoroacetic acid is added, followed by reaction for 1 hour. The reaction system is then adjusted to neutrality and dialyzed with deionized water for 1 day (MWCO 14,000). The product is collected and freeze-dried, yielding Component A-6.1. The labeling of Compound 6 on Hep was identified by 1H NMR. The supernatant of the reaction system was collected, and the residual amount of Compound 6 in the supernatant was determined by HPLC, yielding a labeling rate of approximately 2.56% *(w/w).*

The above structural formulae and embodiments provide examples of the structure and synthesis methods of o-phthalaldehyde-modified heparin-based polymer crosslinkers. Those skilled in the art will be able to conceive of other o-phthalaldehyde-protected derivatives that can be used to synthesize the o-phthalaldehyde-modified heparin-based polymer crosslinkers described in this application.

Embodiment 8: Synthesis of representative Components A-7.1 of o-phthalaldehyde-modified chondroitin sulfate-based crosslinkers

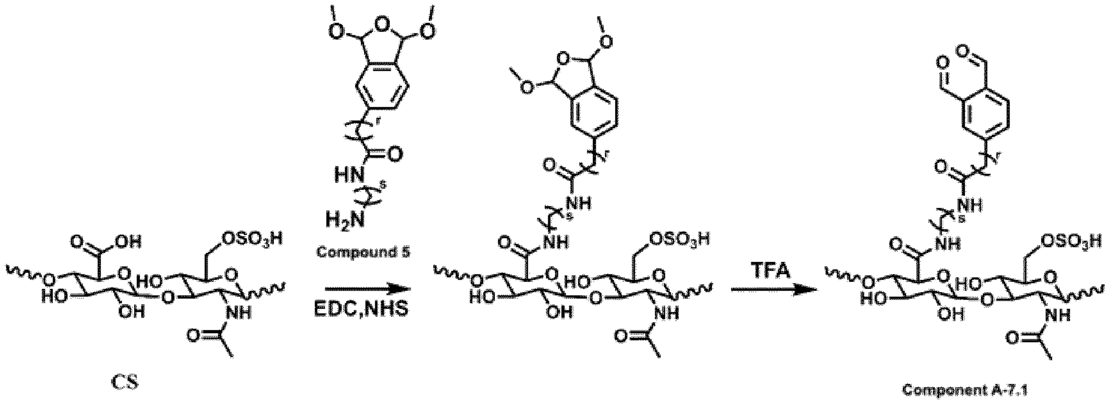


Dissolve chondroitin sulfate (CS, 5 g, 20 kDa) in 50 mL of 0.01 mol/L MES buffer solution (pH = 5.2), stir and sonicate until completely dissolved. Add EDC (1.54 g, 10 mmol) and NHS (1.14 g, 10 mmol) and stir at room temperature for 30 minutes. Dissolve Compound 5 (350 mg, 1 mmol, r = 2, s = 6) in 10 mL of DMSO. Add it to the reaction mixture and stir at room temperature for 24 hours. After the reaction, adjust the pH to 8-9, stir for 1 hour, and dialysis with deionized water for 2-3 days (MWCO 3500). Collect the product and add 10% trifluoroacetic acid, react for 1 hour, then adjust to neutral pH, followed by dialysis of the reaction mixture with deionized water for 1 day (MWCO 3500), collect the product, freeze-dry, and obtain Component A-7.1. The labeling of Compound 5 on CS was identified by 1H NMR. Collect the supernatant from the reaction system, determine the amount of residual Compound 5 in the supernatant by HPLC, and calculate the labeling rate of Compound 5 to be approximately 1.87% (w/w).

The above structural formulae and embodiments provide examples of the structure and synthesis methods of o-phthalaldehyde-modified chondroitin sulfate-based polymer crosslinkers. Those skilled in the art will be able to conceive of other o-phthalaldehyde-protected derivatives that can be used to synthesize the o-phthalaldehyde-modified chondroitin sulfate-based polymer crosslinkers described in this application.

Embodiment 9: Synthesis of representative Components A-8.1 of o-phthalaldehyde-modified xanthan gum-based crosslinkers

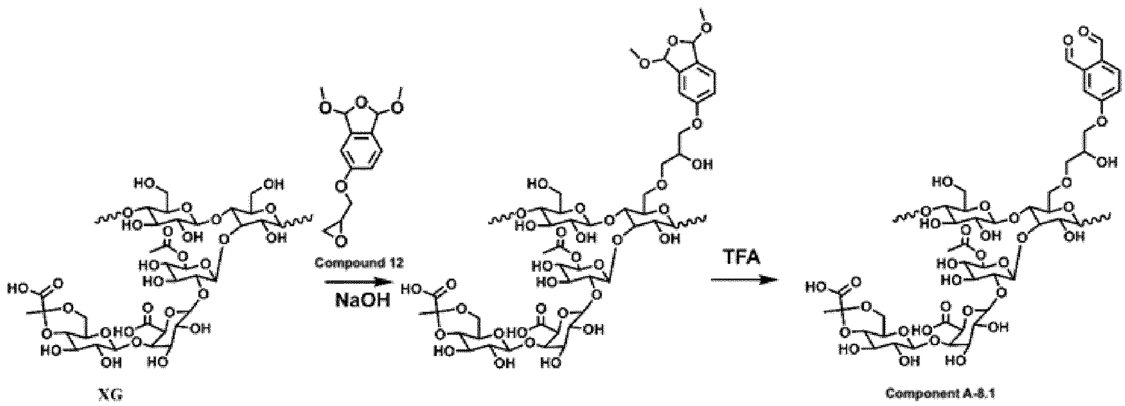


Dissolve xanthan gum (XG, 5 g, 1.2 MDa) in 500 mL of 1% NaOH solution (pH = 5.2) and stir until completely dissolved. Add Compound 12 (302 mg, 1.2 mmol) to the above system and react at 40°C for 12 hours. After the reaction, neutralize the pH to 7 with dilute hydrochloric acid, slowly pour the above system into 10 times the volume of anhydrous ethanol, and collect the white precipitate. The white precipitate was redissolved in 500 mL of deionized water, and 10% trifluoroacetic acid was added. The mixture was reacted for 1 hour, then adjusted to neutral pH. The reaction mixture was dialyzed against deionized water for 1 day (MWCO 14,000), and the product was collected, freeze-dried, yielding Component A-8.1. The labeling of Compound 12 on XG was identified by 1H NMR. Collect the supernatant from the reaction system. Determine the amount of residual Compound 12 in the supernatant by HPLC, and calculate the labeling efficiency of Compound 12 to be approximately 2.10% *(w/w).*

The above structural formulae and embodiments provide examples of the structure and synthesis methods of o-phthalaldehyde-modified xanthan gum-based polymer crosslinkers. Those skilled in the art will be able to conceive of

other o-phthalaldehyde-protected derivatives that can be used to synthesize the o-phthalaldehyde-modified xanthan gum-based polymer crosslinkers described in this application.

Embodiment 10: Synthesis of representative Components A-9.1 of o-phthalaldehyde-modified gellan gum-based crosslinkers

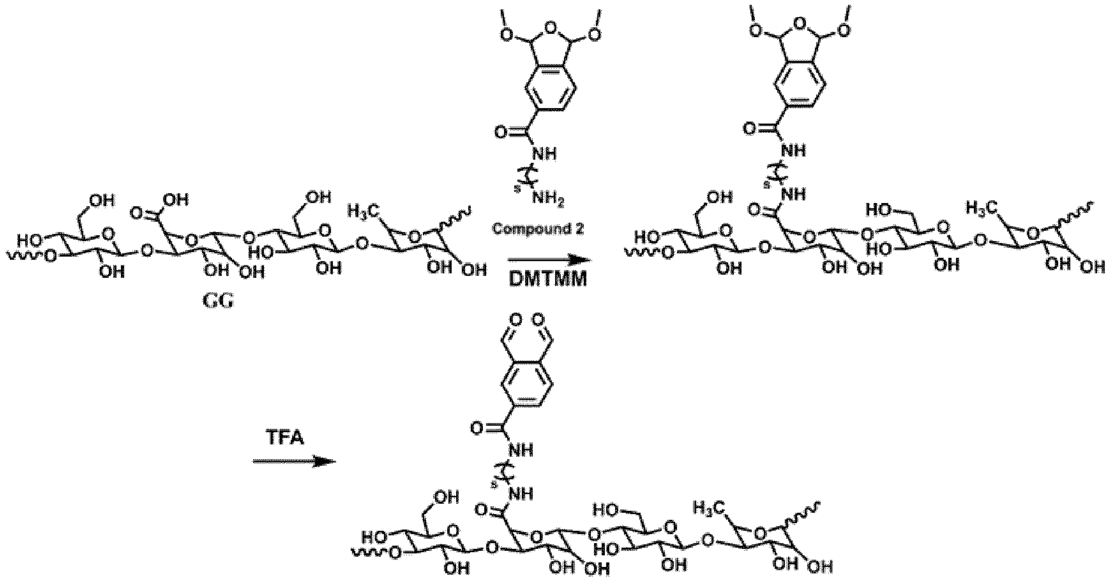


Component A-9.1

Dissolve gellan gum (GG, 5 g, 500 kDa) in 500 mL of 0.01 mol/L MES buffer solution (pH = 5.2), heat to 80°C, and stir until completely dissolved. Dissolve Compound 2 (266 mg, 1 mmol, s = 2) in 10 mL of DMSO, then add to the above reaction mixture. Weigh out DMTMM (7 g, 5 mmol) and add it in five portions to the reaction system, each time dissolved in 3 mL of MES buffer solution, with a 2-hour interval between additions. React at 60°C for 24 hours. After the reaction, adjust the pH to 8-9, stir at 60°C for 1 hour, and add 10 times the volume of anhydrous ethanol during stirring. Collect the precipitate, add 500 mL of deionized water, heat to 60°C, stir until completely dissolved, then add 10% trifluoroacetic acid, react for 1 hour, adjust to neutral pH, then add 10 times the volume of anhydrous ethanol while stirring, collect the precipitate, and vacuum dry to obtain Component A-9.1. The labeling of compound 2 on GG was identified by 1H NMR. Collect the supernatant from the reaction system. Determine the amount of residual Compound 2 in the supernatant by HPLC. The labeling rate of Compound 2 is approximately 1.98% *(w/w).*

The above structural formulae and embodiments provide examples of the structure and synthesis methods of o-phthalaldehyde-modified gellan gum-based polymer crosslinkers. Those skilled in the art will be able to conceive of other o-phthalaldehyde-protected derivatives that can be used to synthesize the o-phthalaldehyde-modified gellan gum-based polymer crosslinkers described in this application.

Embodiment 11: The composition of hydrogels

According to the method of the invention, different polysaccharide-based dual-component hydrogel precursor solutions are prepared (components A and B are separately dissolved in phosphate buffer solution, pH = 7.4).

Table 1

| Component B / Concentration / Component A | Component B-1: Hydrazide-modified hyaluronic acid (340 kDa, labeling rate 4.32%) | Component B-2:bovine serum albumin | Component B-3: gelatin | Component B-... | Component B-7 chitosan (500kDa,90% deacetylation) |
|---|---|---|---|---|---|
| A-1.1 | A：0.1-20 wt% B：0.1-20 wt% | A：0.1-40 wt% B：0.1-40 wt% | A：0.1-40 wt% B：0.1-40 wt% | A：0.1-40 wt% B：0.1-40 wt% | A：0.1-40 wt% B：0.1-40 wt% |
| A-1.2 | A：0.1-20 wt% B：0.1-20 wt% | A：0.1-40 wt% B：0.1-40 wt% | A：0.1-40 wt% B：0.1-40 wt% | A：0.1-40 wt% B：0.1-40 wt% | A：0.1-40 wt% B：0.1-40 wt% |
| A-1.3 | A：0.1-20 wt% B：0.1-20 wt% | A：0.1-40 wt% B：0.1-40 wt% | A：0.1-40 wt% B：0.1-40 wt% | A：0.1-40 wt% B：0.1-40 wt% | A：0.1-40 wt% B：0.1-40 wt% |
| A-1.4 | A：0.1-20 wt% B：0.1-20 wt% | A：0.1-40 wt% B：0.1-40 wt% | A：0.1-40 wt% B：0.1-40 wt% | A：0.1-40 wt% B：0.1-40 wt% | A：0.1-40 wt% B：0.1-40 wt% |
| A-2.1 | A：0.1-20 wt% B：0.1-20 wt% | A：0.1-40 wt% B：0.1-40 wt% | A：0.1-40 wt% B：0.1-40 wt% | A：0.1-40 wt% B：0.1-40 wt% | A：0.1-40 wt% B：0.1-40 wt% |
| A-2.2 | A：0.1-20 wt% B：0.1-20 wt% | A：0.1-40 wt% B：0.1-40 wt% | A：0.1-40 wt% B：0.1-40 wt% | A：0.1-40 wt% B：0.1-40 wt% | A：0.1-40 wt% B：0.1-40 wt% |
| A-2.3 | A：0.1-20 wt% B：0.1-20 wt% | A：0.1-40 wt% B：0.1-40 wt% | A：0.1-40 wt% B：0.1-40 wt% | A：0.1-40 wt% B：0.1-40 wt% | A：0.1-40 wt% B：0.1-40 wt% |
| A-2.4 | A：0.1-20 wt% B：0.1-20 wt% | A：0.1-40 wt% B：0.1-40 wt% | A：0.1-40 wt% B：0.1-40 wt% | A：0.1-40 wt% B：0.1-40 wt% | A：0.1-40 wt% B：0.1-40 wt% |
| A-... | A：0.1-20 wt% B：0.1-20 wt% | A：0.1-40 wt% B：0.1-40 wt% | A：0.1-40 wt% B：0.1-40 wt% | A：0.1-40 wt% B：0.1-40 wt% | A：0.1-40 wt% B：0.1-40 wt% |

| | | | | | |
|---|---|---|---|---|---|
| A-5.1 | A：0.1-20 wt%<br>B：0.1-20 wt% | A：0.1-40 wt%<br>B：0.1-40 wt% | A：0.1-40 wt%<br>B：0.1-40 wt% | A：0.1-40 wt%<br>B：0.1-40 wt% | A：0.1-40 wt%<br>B：0.1-40 wt% |
| A-5.2 | A：0.1-20 wt%<br>B：0.1-20 wt% | A：0.1-40 wt%<br>B：0.1-40 wt% | A：0.1-40 wt%<br>B：0.1-40 wt% | A：0.1-40 wt%<br>B：0.1-40 wt% | A：0.1-40 wt%<br>B：0.1-40 wt% |
| A-6.1 | A：0.1-20 wt%<br>B：0.1-20 wt% | A：0.1-40 wt%<br>B：0.1-40 wt% | A：0.1-40 wt%<br>B：0.1-40 wt% | A：0.1-40 wt%<br>B：0.1-40 wt% | A：0.1-40 wt%<br>B：0.1-40 wt% |
| A-7.1 | A：0.1-20 wt%<br>B：0.1-20 wt% | A：0.1-40 wt%<br>B：0.1-40 wt% | A：0.1-40 wt%<br>B：0.1-40 wt% | A：0.1-40 wt%<br>B：0.1-40 wt% | A：0.1-40 wt%<br>B：0.1-40 wt% |
| A-8.1 | A：0.1-10 wt%<br>B：0.1-10 wt% | A：0.1-10 wt%<br>B：0.1-10 wt% | A：0.1-10 wt%<br>B：0.1-10 wt% | A：0.1-10 wt%<br>B：0.1-10 wt% | A：0.1-10 wt%<br>B：0.1-10 wt% |
| A-9.1 | A：0.1-10 wt%<br>B：0.1-10 wt% | A：0.1-10 wt%<br>B：0.1-10 wt% | A：0.1-10 wt%<br>B：0.1-10 wt% | A：0.1-10 wt%<br>B：0.1-10 wt% | A：0.1-10 wt%<br>B：0.1-10 wt% |

The Components A-... in Table 1 refer to Components A-3.1 to A-4.4 involved in Embodiments 4 to 5; the Components B-... in Table 1 refer to Component B-4: polylysine (5 kDa), Component B-5: collagen, and Component B-6: amino-modified tetra-arm polyethylene glycol (40 kDa). The ranges such as 0.1-40 wt% in Table 1 refer to the preferred mass concentration range of the hydrogel precursor solution.

In the remaining embodiments of the invention, the inventors prepared polysaccharide-based dual-component hydrogels according to the ratios in Table 2 for performance evaluation and applications. The precursor solution and gel state of the polysaccharide-based dual-component hydrogel (Formulation 7) are shown in Figure 3.

Table 2

| No. | Formulation |
|---|---|
| 1 | A-1.2/B-1（1wt%/3wt%） |
| 2 | A-1.2/B-1（2wt%/2wt%） |
| 3 | A-1.2/B-1（2.5wt%/1.5wt%） |
| 4 | A-1.2/B-1（3wt%/1wt%） |
| 5 | A-1.2/B-1（3.5wt%/0.5wt%） |
| 6 | A-1.2/B-2（2wt%/10wt%） |
| 7 | A-1.2/B-3（2wt%/10wt%） |
| 8 | A-1.2/B-7（2wt%/2wt%） |
| 9 | A-1.4/B-1（1wt%/1wt%） |
| 10 | A-1.4/B-2（1wt%/5wt%） |
| 11 | A-1.4/B-3（1wt%/5wt%） |
| 12 | A-1.4/B-7（1wt%/1wt%） |

(continued)

| No. | Formulation |
|---|---|
| 13 | A-2.2/B-1 ( 2wt%/2wt% ) |
| 14 | A-2.2/B-2 (2wt%/10wt% ) |
| 15 | A-2.2/B-3 ( 2wt%/10wt% ) |
| 16 | A-2.2/B-7 ( 2wt%/2wt% ) |
| 17 | A-3.2/B-1 ( 2wt%/2wt% ) |
| 18 | A-3.2/B-2 ( 2wt%/10wt% ) |
| 19 | A-3.2/B-3 ( 2wt%/10wt% ) |
| 20 | A-3.2/B-7 ( 2wt%/2wt% ) |
| 21 | A-4.2/B-1 ( 4wt%/4wt% ) |
| 22 | A-4.2/B-2 ( 4wt%/20wt% ) |
| 23 | A-4.2/B-3 ( 4wt%/20wt% ) |
| 24 | A-4.2/B-7 ( 4wt%/4wt% ) |
| 25 | A-5.1/B-1 ( 1wt%/1wt% ) |
| 26 | A-5.1/B-2 ( 1wt%/5wt% ) |
| 27 | A-5.1/B-3 ( 1wt%/5wt% ) |
| 28 | A-5.1/B-7 ( 1wt%/1wt% ) |
| 29 | A-6.1/B-1 ( 10wt%/2wt% ) |
| 30 | A-6.1/B-2 ( 10wt%/20wt% ) |
| 31 | A-7.1/B-1 ( 10wt%/2wt% ) |
| 32 | A-7.1/B-2 ( 10wt%/20wt% ) |
| 33 | A-8.1/B-1 ( 1wt%/1wt% ) |
| 34 | A-8.1/B-2 ( 1wt%/5wt% ) |
| 35 | A-9.1/B-1 ( 1wt%/1wt% ) |
| 36 | A-9.1/B-2 ( 1wt%/5wt% ) |

Embodiment 12: Hydrogel swelling performance test

To demonstrate that the hydrogel prepared according to the invention has a low swelling property compared to polyethylene glycol-based hydrogels with high swelling (swelling ratio > 100%), the inventors prepared a serious of polysaccharide-based dual-component hydrogels according to the ratios in Table 2 and tested their swelling rates. The o-phthalaldehyde-modified polyethylene glycol crosslinker in Chinese patents CN111440310A, CN111440334A, CN 111574756A, CN 111621038 A, CN113509591A, CN113694249A, CN114767920A, CN202010454896.6, and CN202010455951.3 was selected as the control component A for this invention.

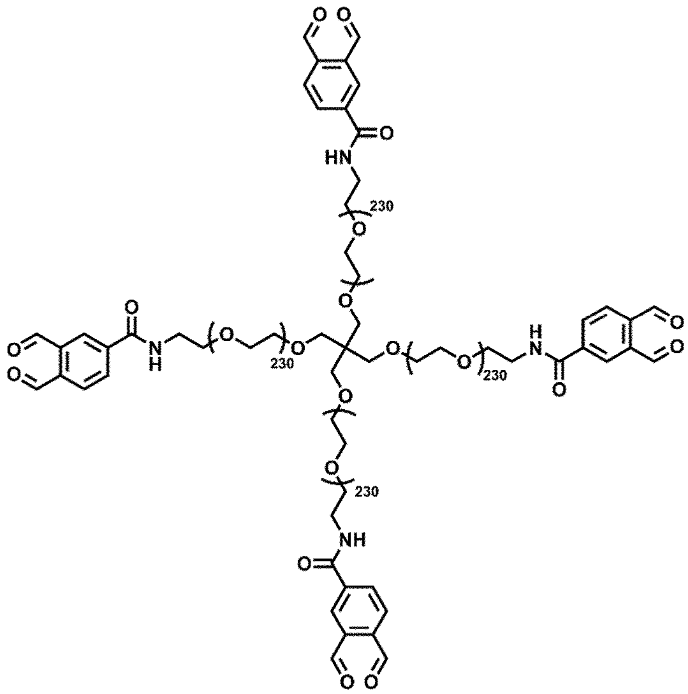

Control component A

Prepare the dual-component hydrogel for the control group according to the ratio in Table 3 for swelling performance test.

Table 3

| No. | Formulation |
|---|---|
| 37 | Control component A/B-1 ( 3wt%/1wt% ) |
| 38 | Control component A/B-2 ( 3wt%/2wt% ) |
| 39 | Control component A /B-3 ( 3wt%/2wt% ) |
| 40 | Control component A /B-7 ( 3wt%/1wt% ) |

The specific testing method is as follows: The solutions of component A and component B from Tables 2 and 3 are extruded into a polytetrafluoroethylene mold using a dual-chamber mixer. After curing for 10 minutes, cylindrical gel blocks with a diameter of 10 mm and a thickness of 3 mm are obtained, all of similar mass. The gel blocks are weighed, and their mass is recorded as *W*0. They are then transferred to a 50 mL centrifuge tube. Add PBS buffer solution with a pH of 7.4 (preheated to 37±1°C) to the centrifuge tube, then place the centrifuge tube in a 37±1°C incubator. Remove the sample every 2 hours, blot the surface moisture with filter paper, and weigh it until the mass no longer increases, recording the mass at this point as *Wt*. Upon completion of the experiment, calculate the gel swelling ratio using the following formula (n = 3):
The swelling ratios obtained from testing by the above method are as shown in the following table: :

$$Swelling\ ratio = \frac{\mathrm{Wt} - \mathrm{W0}}{\mathrm{W0}} \times 100\%$$

The swelling ratios obtained by the above method are shown in the Table 4 below:

Table 4

| No. | Swelling ratio (%) |
|---|---|
| 1 | 35±3.4 |

(continued)

| No. | Swelling ratio (%) |
|---|---|
| 2 | 33±2.1 |
| 3 | 28±3.2 |
| 4 | 35±2.2 |
| 5 | 36±5.4 |
| 6 | 37±3.2 |
| 7 | 31±1.7 |
| 8 | 25±2.7 |
| 9 | 32±3.3 |
| 10 | 24±3.3 |
| 11 | 27±1.2 |
| 12 | 21±1.5 |
| 13 | 26±1.0 |
| 14 | 35±3.2 |
| 15 | 32±2.3 |
| 16 | 17±4.0 |
| 17 | 35±4.8 |
| 18 | 34±2.6 |
| 19 | 19±3.4 |
| 20 | 28±2.7 |
| 21 | 20±3.4 |
| 22 | 22±2.9 |
| 23 | 25±4.3 |
| 24 | 18±2.4 |
| 25 | 27±3.0 |
| 26 | 26±1.9 |
| 27 | 22±4.3 |
| 28 | 22±0.9 |
| 29 | 23±3.2 |
| 30 | 25±2.5 |
| 31 | 16±3.4 |
| 32 | 15±2.0 |
| 33 | 12±2.5 |
| 34 | 18±3.0 |
| 35 | 14±2.6 |
| 36 | 18±2.2 |
| 37 | 105±3.2 |
| 38 | 112±2.9 |
| 39 | 106±4.3 |
| 40 | 120±1.5 |

In the above formulations, compared with the polyethylene glycol-based hydrogels in the control group (i.e., Formulations 37-40), the polysaccharide-based hydrogels of the present invention have low swelling rates (<50%) over a wide range of solid contents.

Embodiment 13: Hydrogel tissue adhesion performance test

The inventors conducted tissue adhesion performance tests on the polysaccharide-based dual-component hydrogel of the invention and selected commercially available fibrin glue as the control group. The specific testing method is as follows: Using a tensile testing machine (INSTRON), the shear strength of the aforementioned different formulations of hydrogel on pig skin was measured through standard lap shear tests (ASTM F2255). Pig skin was cut into rectangular pieces measuring 10 mm and 40 mm. During mechanical testing, transparent polymethyl methacrylate (PMMA) film was used as a rigid backing for the tissue. The dual-component solutions of Component A and Component B from Table 2 or the control fibrin glue were sprayed onto a piece of pig skin using a dual-chamber mixer, covering an area of 15 mm × 10 mm, i.e., the adhesion area. Immediately press another piece of pig skin onto the pig skin coated with the hydrogel precursor solution, applying uniform pressure at 100 kPa for 10 minutes. After allowing the hydrogel formulations to cure completely, perform tensile testing. Adhesion strength is calculated using the following formula, where *Fmax* represents the maximum force value. To ensure data reliability, measurements were taken on three samples under each condition.

$$Adhesion\ strength = \frac{\mathrm{Fmax}}{\mathrm{Adhesion\ area}}$$

The adhesion strength of the hydrogel obtained by the above method is shown in Table 5:

Table 5

| No. | Adhesion strength (kPa) |
|---|---|
| 1 | 18.45±2.23 |
| 2 | 37.76±4.65 |
| 3 | 45.78±4.86 |
| 4 | 66.54±3.22 |
| 5 | 77.25±4.54 |
| 6 | 62.22±4.12 |
| 7 | 58.15±5.87 |
| 8 | 55.64±5.23 |
| 9 | 47.32±5.21 |
| 10 | 52.28±4.56 |
| 11 | 50.89±4.21 |
| 12 | 54.32±8.41 |
| 13 | 46.56±4.33 |
| 14 | 69.22±6.21 |
| 15 | 52.21±5.98 |
| 16 | 44.32±5.55 |
| 17 | 34.79±4.23 |
| 18 | 52.87±4.95 |
| 19 | 49.12±3.46 |
| 20 | 49.86±5.27 |
| 21 | 40.68±4.92 |
| 22 | 63.53±5.20 |

(continued)

| No. | Adhesion strength (kPa) |
|---|---|
| 23 | 52.52±4.35 |
| 24 | 33.18±4.10 |
| 25 | 40.55±9.13 |
| 26 | 45.97±21.34 |
| 27 | 46.65±7.60 |
| 28 | 38.27±5.42 |
| 29 | 38.67±6.25 |
| 30 | 60.53±10.43 |
| 31 | 38.95±6.80 |
| 32 | 24.25±5.32 |
| 33 | 32.33±7.85 |
| 34 | 38.25±3.92 |
| 35 | 40.89±9.28 |
| 36 | 34.54±6.55 |
| Fibrin glue | 13.24±1.18 |

The test results in Table 5 show that, compared with commercially available fibrin glue, the polysaccharide-based dual-component hydrogel of the invention exhibits excellent tissue adhesion across a wide range of solid content, enabling tissue bonding or sealing applications. In Formulations 1 to 5, as the proportion of Component A increases, the tissue adhesion of the polysaccharide-based dual-component hydrogel improves. It is evident that increasing the proportion of Component A in the polysaccharide-based dual-component hydrogel effectively enhances the hydrogel's tissue adhesion.

Embodiment 14: In vitro hemostatic performance testing of hydrogels

The inventors conducted in vitro hemostatic performance tests on the polysaccharide-based dual-component hydrogel of the invention and selected commercially available fibrin glue as the control group. The specific testing method is as follows: The solutions of Component A and Component B from Table 2, or the dual-component solution of the control group Fibrin glue, were sprayed onto a clean 96-well plate using a dual-channel dispenser, allowed to solidify for 10 minutes, and then incubated at 37°C. Add 20 μL of fresh EDTA-anticoagulated sheep blood at 37°C to the material surface. For the blank control group, directly add the blood to the bottom of the 96-well plate and incubate at 37°C for 5 minutes. Subsequently, the blood or thrombus on the surface of the hydrogel is washed with a total of 1 mL of deionized water and gently shaken. The size of the remaining blood clots on the hydrogel surface is observed. The supernatant is aspirated, and the absorbance at 540 nm is measured. The thrombus formation index is calculated using the formula below. The results are shown in Table 6.

$$Blood\ clotting\ index = \frac{100 \times \text{Sample absorbance at 540 nm}}{\text{Blank absorbance at 540 nm}}$$

The blood clotting index for hydrogels determined using the above method is shown in Table 6 :

Table 6

| No. | Blood clotting index |
|---|---|
| 1 | 0.53 |
| 2 | 0.42 |
| 3 | 0.22 |

(continued)

| No. | Blood clotting index |
|---|---|
| 4 | 0.10 |
| 5 | 0.05 |
| 6 | 0.12 |
| 7 | 0.14 |
| 8 | 0.12 |
| 9 | 0.14 |
| 10 | 0.08 |
| 11 | 0.13 |
| 12 | 0.09 |
| 13 | 0.23 |
| 14 | 0.20 |
| 15 | 0.19 |
| 16 | 0.14 |
| 17 | 0.30 |
| 18 | 0.22 |
| 19 | 0.23 |
| 20 | 0.16 |
| 21 | 0.24 |
| 22 | 0.09 |
| 23 | 0.20 |
| 24 | 0.16 |
| 25 | 0.32 |
| 26 | 0.21 |
| 27 | 0.26 |
| 28 | 0.19 |
| 29 | 0.17 |
| 30 | 0.11 |
| 31 | 0.26 |
| 32 | 0.14 |
| 33 | 0.17 |
| 34 | 0.10 |
| 35 | 0.14 |
| 36 | 0.10 |
| Fibrin glue | 0.43 |

The test results in Table 6 show that, compared with commercially available fibrin glue, the polysaccharide-based dual-component hydrogel of the invention exhibits excellent hemostatic performance across a wide range of solid content levels, making it suitable for hemostatic applications. In Formulations 1 to 5, as the proportion of Component A increases, the in vitro hemostatic performance of the polysaccharide-based dual-component hydrogel improves. It is evident that increasing the proportion of Component A in the polysaccharide-based two-component hydrogel effectively enhances the hemostatic efficacy of the hydrogel.

Embodiment 15: Based on the product formulations obtained from the above embodiments, and taking into consideration swelling properties, tissue adhesion, and hemostatic performance, Formulations 5, 6, 7, 10, 14, 22, and 30 of dual-component hydrogels were selected for further implementation in specific biomedical applications.

Embodiment 16: Polysaccharide-based dual-component hydrogel for skin wound repair

SD rats were used for skin defect wound repair experiments, divided into two groups: Formulation 5 (Group A) and suture group (Group B). A 10 mm skin defect wound was created on the back of SD rats. For Group A, Solutions A and B were injected into the skin defect site using a dual-chamber mixer, allowed to fully fill and penetrate the wound, and cured for 10 minutes, resulting in the hydrogel firmly adhering to the wound surface. For Group B, the defect site was sutured using 4-0 non-absorbable surgical suture thread. The wound surfaces of each group were observed, and the repair effects were recorded over 7 days. The experimental results showed that compared to Group B, Group A exhibited faster repair rates and no significant scar tissue formation. In this example, photographs of the skin tissue repair at 7 days using the polysaccharide-based dual-component hydrogel (Group A) and suture (Group B) are shown in Figure 4. It can be seen that the aforementioned polysaccharide-based dual-component hydrogel can be applied to skin wound repair.

The polysaccharide-based dual-component hydrogels of other formulations in this invention can also be applied to skin wound repair.

Embodiment 17: Polysaccharide-based dual-component hydrogel for abdominal wall wounds repair

The SD rat abdominal wall abrasion model was selected, and the abdominal wall wound repair experiment was conducted in two groups: Formulation 5 (Group A) and the control group (Group B). SD rats were anesthetized and restrained, their abdominal hair was shaved off to expose the abdominal cavity, and the surgical blade was used to scrape the left and right sides of the abdominal wall several times until bleeding occurred, resulting in an abdominal wall abrasion model with a diameter of approximately 10 mm. Solutions of Component A and Component B were injected into the abdominal wall abrasion site using a dual-chamber mixer, allowed to fully fill and penetrate the wound, and cured for 10 minutes. In Group B, the abdominal wall wound was cleaned with physiological saline. The wounds and hydrogels were observed, and the repair effects of each group were recorded after 7 days. The experimental results showed that the hydrogel in Group A firmly adhered to the wound surface. Compared to Group B, the abdominal wall wound treated with Group A hydrogel showed no significant adhesion to the abdominal organs and exhibited a faster repair rate. Thus, the aforementioned polysaccharide-based dual-component hydrogel can be applied to abdominal wall wound repair.

The polysaccharide-based dual-component hydrogels of other formulations in this invention can also be applied to abdominal wall wound repair.

Embodiment 18: Polysaccharide-based dual-component hydrogel for sealing pancreatic fluid leakage

The SD rat pancreatic fistula model was selected, and the pancreatic fistula leakage sealing experiment was conducted in two groups: Formulation 5 (Group A) and the control group (Group B). SD rats were anesthetized and immobilized, their abdominal hair was shaved, and the area was disinfected with iodine solution. The abdominal cavity was exposed, the omentum and pancreas were separated, and part of the spleen vessels were ligated to prevent unnecessary bleeding during pancreas resection. The distal end of the pancreas-duodenum was resected with a surgical knife to create the pancreatic leakage model. For Group A, Solutions A and B were injected at the pancreatic stump using a dual-chamber syringe, allowed to fully fill and penetrate the wound, and cured for 10 minutes; Group B received no treatment and the abdomen was closed directly. On day 7 post-surgery, collect peritoneal fluid from SD rats and analyze its protease content; on day 7 post-surgery, euthanize SD rats and observe peritoneal adhesion. Experimental results show that protease levels in SD rats in Group B exceeded normal levels within 7 days post-surgery, confirming the successful establishment of the pancreatic fistula model. In this example, the polysaccharide-based dual-component hydrogel (Formulation 5) was applied to the pancreatic stump to block pancreatic fluid leakage. The photograph of the abdominal cavity 7 days postoperatively is shown in Figure 5. Compared to Group B, Group A rats showed no significant ascites or adhesions in the abdominal cavity, and the hydrogel material could still be observed at the pancreatic stump. After flushing the abdominal cavity with physiological saline, the accumulated fluid was collected for protease level detection. The experimental results showed that the pancreatic protease content in the abdominal cavity of Group A rats was within normal levels. Thus, the aforementioned polysaccharide-based dual-component hydrogel can be applied for pancreatic fluid leakage sealing.

The polysaccharide-based dual-component hydrogels of other formulations in this invention can also be applied for pancreatic fluid leakage sealing.

Embodiment 19: Polysaccharide-based dual-component hydrogel for sealing cerebrospinal fluid leaks

The SD rat cerebrospinal fluid leakage model was selected, and cerebrospinal fluid leakage sealing experiments were conducted in two groups: Formulation 5 (Group A) and bone wax group (Group B). Anesthetize and immobilize the SD rats, shave the hair on the back, and make incisions through the skin and fascia centered on T13 and L1 to expose the surgical area muscles. On one side, use ophthalmic scissors to cut along the spinous process, severing the connected muscles and tendons, creating an incision with a length of approximately T12 to L2 and a depth reaching the articular process. Make another parallel incision approximately 0.5 cm lateral to the first incision, with similar length and depth. Remove the muscles between the two incisions to expose three pairs of articular processes (T12-T13, T13-L1, and L1-L2). Expose the lateral walls of the spinal canal and the intervertebral foramen, then parallelly transect the lateral walls of the spinal canal to expose the spinal cord, creating a wound surface. If bleeding and cerebrospinal fluid leakage are observed, the modeling is considered successful. Subsequently, for Group A, the Solution A and Solution B were immediately injected into the cerebrospinal fluid leakage site using a dual-chamber syringe, allowing the solution to fully fill and penetrate the wound, followed by curing for 10 minutes; for Group B, bone wax was immediately applied to the cerebrospinal fluid leakage site, but leakage persisted during use in Group B. In this example, photographs of the application of the polysaccharide-based two-component hydrogel (Formulation 5, Group A) and bone wax (Group B) for cerebrospinal fluid leakage sealing are shown in Figure 6. The wounds of both groups of rats were sutured. At 2 days post-surgery, all rats in Group B died, while all rats in Group A survived; at 7 days post-surgery, the hydrogel was observed to be firmly adhered to the spinal injury site in Group A rats, with no significant leakage. It is evident that the aforementioned polysaccharide-based two-component hydrogel can be applied for cerebrospinal fluid leakage sealing.

The polysaccharide-based dual-component hydrogels of other formulations in this invention can also be applied for cerebrospinal fluid leakage sealing.

Embodiment 20: Polysaccharide-based dual-component hydrogel for sealing air leaks in lung parenchyma

A New Zealand white rabbit lung parenchymal air leak model was selected, and lung parenchymal air leak occlusion experiments were conducted in two groups, each consisting of three rabbits: Formulation 6 (Group A) and the control group (Group B). New Zealand white rabbits were anesthetized via ear vein injection of 1% pentobarbital sodium at a dose of 3 mL/kg, followed by tracheal intubation and mechanical ventilation (VT 80 mL, R 30 bpm). Thoracotomy was then performed, and the lung lobe margin tissue was resected, creating a wound area of approximately 1 $cm^2$, with at least one visible bronchiole stump of approximately 1 mm in diameter. For Group B, the wound was not treated, and the chest was closed directly. For Group A, Solutions A and B were injected into the lung defect site using a dual-chamber syringe, allowed to fully fill and penetrate the wound, and cured for 10 minutes. After confirming that the hydrogel adhered to the wound site, the chest was closed layer by layer, residual air was expelled, and the lung was fully re-expanded. In Group B, three rabbits died from respiratory failure due to persistent air leakage within 4 hours postoperatively. In Group A, no air leakage was observed at the lung wound site. At 7 and 14 days postoperatively, postmortem examination revealed that the hydrogel material in Group A was tightly adhered to the wound site, with no fluid accumulation or adhesion observed. Thus, the aforementioned polysaccharide-based two-component hydrogel can be applied for sealing air leaks in lung parenchyma.

The polysaccharide-based dual-component hydrogels of other formulations in this invention can also be applied for sealing air leaks in lung parenchyma.

Embodiment 21: Polysaccharide-based dual-component hydrogel for liver hemostasis

The SD rat liver transverse section trauma model was selected, and liver hemostasis experiments were conducted in three groups: Formulation 14 (Group A), Formulation 7 (Group B), and the gauze group (Group C). After anesthetizing the SD rats, their abdominal cavities were opened, the livers were lifted and placed on pre-weighed filter paper, and a 10-millimeter transverse section was created on the liver to induce bleeding. For Groups a and b, solutions of Component A and Component B were injected at the bleeding site using a dual-chamber syringe. As the solutions penetrated, Groups A and B rapidly gelled in the presence of blood, sealing the bleeding site. For Group C, pre-weighed gauze was gently pressed onto the transverse wound surface. Blood loss was measured by the weight gain of the filter paper (and gauze), and hemostasis time was recorded using a timer. During the experiment, compared to Group C, Groups A and B showed a significant reduction in blood loss (Group A: 0.2 g, Group B: 0.1 g, Group C: 1.0 g) and a marked shortening of hemostasis time (Group A: 7 s, Group B: 5 s, Group C: 50 s). Thus, the aforementioned polysaccharide-based dual-component hydrogel can be applied for liver hemostasis.

The polysaccharide-based dual-component hydrogels of other formulations in this invention can also be applied for liver hemostasis.

Embodiment 22: Polysaccharide-based dual-component hydrogel applied to femoral artery hemostasis

The SD rat femoral artery bleeding model was selected, and femoral artery hemostasis experiments were conducted in three groups: Formulation 14 (Group A), Formulation 10 (Group B), and the gauze group (Group C). After anesthetizing the SD rats, the hair on the right hind leg and lower abdomen was shaved to expose the femoral artery, vein, and nerves. The femoral artery was carefully dissected, and hemostatic forceps were used to gently clamp both ends of the artery. A 32 G needle was then used to puncture the artery. For Groups A and B, after the arterial blood had completely drained, the solutions of Component A and Component B were immediately injected into the bleeding site using a dual-chamber mixer; for Group C, gauze was immediately applied and gently pressed onto the bleeding site. The experimental results showed that Groups A and B rapidly gelled in the presence of blood. After removing the hemostatic forceps from both ends, the hydrogel remained firmly adhered to the bleeding site, achieving stable occlusion and preventing further bleeding. In Group C, the rats died from excessive blood loss and failed to achieve hemostasis. It is evident that the aforementioned polysaccharide-based two-component hydrogel can be applied for femoral artery hemostasis.
The polysaccharide-based dual-component hydrogels of other formulations in this invention can also be applied for femoral artery hemostasis.

Embodiment 23: Preparation of polysaccharide-based membrane materials

Taking Formulation 5 as an example, components A and B are dissolved separately in phosphate buffer solution (pH = 7.4) to obtain component A solution and component B solution. The two solutions are then applied to a clean, flat polytetrafluoroethylene plate using a dual-chamber mixing device. After curing for 10 minutes, the material is swollen to equilibrium in a phosphate-buffered solution containing 5% glycerol (pH=7), dried at a constant temperature of 30°C, and the polysaccharide-based membrane material is obtained.
The precursor solutions of other formulations in this invention can also be used to prepare polysaccharide-based membrane materials with different compositions.

Embodiment 24: Polysaccharide-based membrane material used for sutureless closure of surgical wounds

The SD rat dorsal wound model was selected, and two groups were established for a sutureless wound closure experiment: the membrane material group (Formulation 5, Group A) and the suture group (Group B) as described in Example 23. After anesthetizing the SD rats, their dorsal hair was shaved, and a 1 cm-long skin incision was made on their backs using a surgical knife. For Group A, the membrane material was applied to the incision site to close the wound. For Group B, the incision was aligned and sutured using 4-0 non-absorbable surgical suture thread. The healing status of the incision was observed at 3 days and 10 days post-surgery: In Group A, the membrane material showed no significant detachment, with rapid epithelialization of the incision and fast healing speed; in Group B, the healing speed was slow. In this example, the polysaccharide-based membrane material was applied to surgical wounds for suture-free closure (Group A) and suturing (Group B). The H&E staining results at 10 days post-surgery are shown in Figure 7. The H&E staining results indicate that the wound in Group A healed completely, with new tissue resembling skin tissue, while the wound in Group B was not fully repaired. Thus, the aforementioned polysaccharide-based membrane material can be applied for sutureless closure of surgical wounds.
The polysaccharide-based dual-membrane materials of other formulations in this invention can also be applied for sutureless closure of surgical wounds.

Embodiment 25: Polysaccharide-based membrane material as patches for abdominal hernia repair

An acute abdominal wall hernia model in SD rats was selected, and the abdominal wall hernia repair experiment was conducted in two groups: the membrane material from Embodiment 23 (Formulation 5, Group A) and the Separmesh absorbable composite patch (Bard, Group B). After anesthetizing the rats, they were immobilized, shaved, and disinfected. A 1.2 cm × 1.2 cm square was marked on each side of the midline of the lower abdomen, 2 cm from the midline. The abdominal wall was incised layer by layer to create a 1.2 cm diameter full-thickness abdominal wall defect. A 2 cm × 2 cm square patch was placed on each side. The patch in Group A was not sutured; the patch in Group B was intermittently sutured around the edges using 4-0 non-absorbable surgical suture. The skin was disinfected with 75% ethanol and then intermittently sutured using 3-0 non-absorbable surgical suture. Thirty days postoperatively, the abdominal cavity was opened to observe the adhesion between the abdominal organs and the patch. The experimental results showed that in Group A, the patches did not shift significantly, no intra-abdominal hernias occurred, and there was no significant adhesion between the patches and the abdominal organs; in Group B, the patches did not shift significantly, but there was slight adhesion between the suture sites and the greater omentum in the abdominal cavity. It can be seen that the aforementioned polysaccharide-based membrane materials can be used as patches for abdominal hernia repair.

The polysaccharide-based dual-membrane materials of other formulations in this invention can also be applied for abdominal hernia repair.

Embodiment 26: Preparation of polysaccharide-based powder materials

Taking Formulation 22 as an example, components A and B are dissolved separately in phosphate buffer solution (pH = 7.4) to obtain component A solution and component B solution. The two solutions are then applied to a clean, flat polytetrafluoroethylene plate using a dual-chamber mixing device. After curing for 10 minutes, the material is swollen to equilibrium in phosphate buffer solution (pH=7), dried at a constant temperature of 30°C, and then ground in a ball mill. After sieving, a fine powder (mesh size 60-300) is obtained, which is the polysaccharide-based powder material. In this embodiment, the microscopic morphology of polysaccharide-based powder materials with different particle sizes under a microscope is shown in Figure 8. Alternatively, the cured hydrogel material is mixed with an equal volume of phosphate-buffered solution (pH=7), and high-speed shear mixing is used to cut the hydrogel blocks into smaller microgel particles. Subsequent to the separation of the microgels, three times the volume of 50%, 75%, and anhydrous ethanol are added in sequence to replace the water in the microgels. The microgels are separated, dried under vacuum at 30°C for 12 hours, sieved, and ground into a powder with a mesh size of 60-300 mesh, yielding the polysaccharide-based powder material.

The precursor solutions of other formulations in this invention can also be used to prepare polysaccharide-based powder materials with different compositions.

Embodiment 27: Polysaccharide-based powder material used for hemostasis in minimally invasive surgery

An acute upper gastrointestinal arterial bleeding model using Baima pigs was selected, and minimally invasive surgical hemostasis experiments were conducted in two groups: the powdered material from Embodiment 26 (Formulation 22, Group A) and absorbable hemostatic particles (ARISTA, Group B). Adult female Baima pigs (weight 20-25 kg) were anesthetized, and a catheter was inserted into the right femoral artery to monitor blood pressure. Within 5 minutes, baseline blood pressure (systolic pressure) was monitored in all animals and adjusted to 90-100 mm Hg, followed by intravenous administration of heparin (10 mg/kg) to achieve systemic heparinization. Under endoscopic guidance, the mucosa was peeled off, and the submucosal pulsating artery in the stomach was identified. The artery was then cut with an endoscopic knife to induce acute upper gastrointestinal bleeding, and natural bleeding was maintained for 4 minutes. The hemostatic powders from Group A and Group B were applied to the bleeding site via the catheter. The bleeding site was observed, and the hemostasis procedure was repeated until hemostasis was achieved, with hemostasis time and material quality recorded. The experimental results showed that during the procedure, the hemostatic powder in Group A rapidly adhered to the wound surface and formed a gel-like substance to seal the bleeding, resulting in a shorter hemostasis time (Group A: 80 s, Group B: 160 s) and less material used (Group A: 1.1 g, Group B: 2 g). This indicates that the aforementioned polysaccharide-based powder material can be applied for hemostasis in minimally invasive surgery.

The polysaccharide-based powder materials of other formulations in this invention can also be applied for minimally invasive surgery.

Embodiment 28: Polysaccharide-based powder materials as antimicrobial drug carriers for wound antimicrobial treatment

Taking Formula 14 as an example, in this example, components A and B are dissolved in phosphate buffer solution (pH = 7.4) to obtain component A solution and component B solution, respectively. The two solutions are then applied to a clean, flat polytetrafluoroethylene plate using a dual-chamber mixing device. After curing for 10 minutes, an equal volume of phosphate buffer solution (pH = 7) is added. and a high-speed shear mixer is used to cut the block-shaped hydrogel into microgels with smaller particle sizes. The microgels are dispersed in a phosphate-buffered solution (pH=7.4) containing benzalkonium chloride (0.1%), and stirred for 1 hour. Due to electrostatic adsorption, the cationic benzalkonium chloride adsorbs onto the surface of the microgels, after separating the microgels, add 3 volumes of 50%, 75%, and anhydrous ethanol, respectively, to replace the water in the microgels. Separate the microgels, dry them under vacuum at 30°C for 12 hours, sieve them, and obtain the benzalkonium chloride-loaded polysaccharide-based powder material.

A dorsal full-thickness incision wound infection model was used in Balb/C mice, and three groups were conducted for topical wound antimicrobial experiments: the aforementioned surface-loaded benzalkonium chloride-containing polysaccharide-based powder material group (Group A), the benzalkonium chloride solution group (Group B), and the control group (Group C). A20 mm full-thickness longitudinal incision was made on the back of 8- to10-week-old female BALB/C mice, and 50 μL of Escherichia coli 25922 ($1\times10^8$ CFU mL-1) was dropped onto the wound site to establish an in situ acute pathogenic bacterial infection model. Group A powder was evenly sprayed onto the wound site, where it gradually gelled and firmly adhered to the wound surface as wound exudate was released; Group B wounds were disinfected with 0.01% benzalkonium chloride solution, and Group C wounds received no treatment. The experimental

results showed that in Group C, the infected area began to spread, with impaired blood circulation, gradually leading to ischemia and suppuration, and significant tissue damage; in Group B, the initial spread of the infected area was limited, but suppuration and ischemia still occurred by day 7 post-surgery; Group A showed the best recovery, with bacterial proliferation inhibited and no suppuration or ischemia at the wound site. It is evident that the aforementioned polysaccharide-based powder material loaded with benzalkonium chloride can be applied for wound antibacterial treatment.

The polysaccharide-based powder materials of other formulations in this invention can also be applied for wound antibacterial treatment.

Embodiment 29: Polysaccharide-based powder materials as carriers for antiinflammatory drugs in the treatment of inflammation

Glucocorticoid drugs have a wide range of indications and exert strong regulatory effects on various physiological functions throughout the body. They are widely used in clinical practice for various autoimmune diseases, leukemia, asthma, allergic reactions, and other conditions. However, prolonged non-specific use of glucocorticoid drugs may induce various diseases and have severe side effects. Therefore, local, short-term, low-dose use of glucocorticoid drugs is the recommended method of administration in clinical practice. Taking Formulation 5 as an example, in the embodiment, components A and B are dissolved in phosphate-buffered solution (pH=7.4) to obtain component A solution and component B solution, respectively. The two solutions are then applied to a clean, flat polytetrafluoroethylene plate using a dual-chamber mixer, and allowed to cure for 10 minutes. add an equal volume of phosphate-buffered solution (pH=7), and use high-speed shear mixing to cut the block-shaped hydrogel into smaller microgel particles. Disperse the microgels in phosphate-buffered solution (pH = 7.4) containing budesonide (20 μg/mL) and stir for 1 hour. Due to hydrogen bonding and hydrophilic-hydrophobic interactions, budesonide adsorbs onto the microgel surface. After separating the microgels, sequentially add three times the volume of 50%, 75%, and anhydrous ethanol to replace the water in the microgels. The microgels were separated, dried under vacuum at 30°C for 12 hours, sieved, and the resulting polysaccharide-based powder material loaded with budesonide was obtained.

An acute enteritis model using Balb/C mice was selected, and acute enteritis treatment experiments were conducted in three groups: the aforementioned surface-loaded budesonide polysaccharide powder material (group A), budesonide enema solution (group B), and a blank group (group C). A 3% DSS drinking water solution was prepared using sterile water and filtered through a 0.22 μm filter membrane. The mice were administered the solution continuously for 7 days. Colonoscopy revealed localized edema and congestion in the mouse tissues, with obvious inflammatory phenomena, indicating successful modeling. Group A applied the powdered material to the inflamed area via endoscopy, Group B administered budesonide enema solution via enema, and Group C received no treatment. After 7 and 14 days, the condition of the inflamed areas was observed, and the colon lengths of mice in each group were measured and statistically analyzed. The experimental results showed that the powdered material in Group A firmly adhered to the damaged inflammatory wound surface, and compared to Groups B and C, the edema and ulceration of the wound surface were alleviated. In this example, the statistical graph of colon length in mice treated with normal mice, polysaccharide-based powdered material loaded with budesonide (Group A), budesonide enema solution (Group B), and the control group (Group C) for acute colitis is shown in Figure 9. Statistical results indicate that the colon length of mice in Group A was the longest compared to Groups B and C. It is evident that the aforementioned budesonide-loaded polysaccharide-based powdered material can be applied to inflammatory treatment.

The polysaccharide-based powder materials of other formulations in this invention can also be applied to inflammatory treatment.

Embodiment 30: Preparation of polysaccharide-based sponge materials

Taking Formulation 6 as an example, solutions A and B are obtained by dissolving components A and B separately in phosphate buffer solution (pH = 7.4). The two solutions are sprayed into a mold using a dual-chamber mixer, cured for 10 minutes, swollen to equilibrium in phosphate buffer solution (pH = 7), frozen at -20°C for 6 hours, and then freeze-dried at -60°C for 48 hours to obtain a polysaccharide-based sponge material.

The precursor solutions of other formulations in this invention can also be used to prepare polysaccharide-based sponge materials with different compositions.

Embodiment 31: Polysaccharide-based sponge materials are used as wound dressings in wound treatment.

The SD rat dorsal defect model was selected, and wound dressing experiments were conducted in two groups: the sponge material from Example 30 (Formulation 14, Group A), the commercial gelatin sponge group (Group B), and the control group (Group C). A circular defect wound with a diameter of 10 mm was created on the back of SD rats. The sponge materials from Group A and Group B were uniformly applied to the wound, while Group C received no treatment. Subsequently, all groups were covered with gauze and medical adhesive tape. The experimental results showed that the sponge material in Group a could firmly adhere to the tissue defect site. Compared to Group C, Groups A and B exhibited

faster wound healing rates, with newly formed tissue resembling skin tissue and no significant scar tissue formation. Thus, the aforementioned polysaccharide-based sponge material can be applied as a wound dressing for wound treatment.

The polysaccharide-based sponge materials of other formulations in this invention can also be applied as a wound dressing for wound treatment.

Embodiment 32: Polysaccharide-based sponge materials are used for spleen hemostasis.

A New Zealand white rabbit spleen hemorrhage model was selected, and spleen hemostasis experiments were conducted in two groups: the sponge material from Embodiment 30 (Formulation 6, Group A) and commercially available absorbable hemostatic gauze (Group B). New Zealand white rabbits were anesthetized and restrained, their abdominal hair was shaved, the abdominal cavity was disinfected with iodine solution, and the spleen was exposed. Using a surgical knife, an 8mm-long, 1mm-deep incision was made on the spleen to create a bleeding wound. The hemostatic material was gently pressed onto the bleeding site, and the wound was observed approximately every 10 seconds. Once bleeding had completely stopped, the hemostasis time was immediately recorded. In this example, photographs comparing the application of polysaccharide-based sponge material (Group A) and commercial absorbable hemostatic gauze (Group B) for splenic hemostasis are shown in Figure 10. The hemostatic materials were weighed before and after use, and the difference represented the total blood loss. During the experiment, both Group A and Group B materials adhered firmly to the bleeding wound on the spleen. Compared to Group B, Group A had a shorter hemostasis time (Group A: 120 s, Group B: 180 s) and less blood loss (Group A: 3 g, Group B: 3.5 g). It is evident that the aforementioned polysaccharide-based sponge material can be applied for spleen hemostasis.

The polysaccharide-based sponge materials of other formulations in this invention can also be applied for spleen hemostasis.

Embodiment 33: Polysaccharide-based sponge materials are used for cardiac hemostasis.

The SD rat cardiac hemorrhage model was selected, and cardiac hemostasis experiments were conducted in two groups: the sponge material group (Formulation 30, Group A) and the gauze group (Group B) from Embodiment 30. SD rats were anesthetized and restrained, and the chest hair was shaved. The thoracic cavity was opened to expose the heart, and the pericardium was removed using fine forceps. A biopsy punch was used to create a 2 mm diameter injury on the left or right ventricular wall of the heart. The hemostatic material was immediately applied to the bleeding site and subjected to the same pressure. The wound was observed approximately every 10 seconds, and the hemostasis time was recorded immediately after bleeding ceased completely. In this example, photographs of the polysaccharide-based sponge material (Group A) applied for cardiac hemostasis are shown in Figure 11. The hemostatic material was weighed before and after use, and the difference represented the total blood loss. For groups that failed to achieve hemostasis within 300 seconds, the rats were euthanized by bleeding. During the experiment, the sponge in Group A firmly adhered to the ventricular wound surface (as shown in Figure 11). Compared to Group B, Group A had a shorter hemostasis time (Group A: 60 s; Group B rats failed to achieve hemostasis within 300 s) and the least blood loss (Group A: 1.2 g; Group B lost 2.5 g of blood within 300 s), and the rats in Group A successfully survived postoperatively. It is evident that the aforementioned polysaccharide-based sponge material can be applied for cardiac hemostasis.

The polysaccharide-based sponge materials of other formulations in this invention can also be applied for cardiac hemostasis.

Embodiment 34: Polysaccharide-based sponge material applied to non-compression hemostasis for penetrating wounds.

A New Zealand white rabbit liver perforation model was selected, and two groups were established for non-compressive penetrating wound hemostasis experiments: the sponge material group (Formulation 6, Group A) and the gauze group (Group B) from Embodiment 30. After anesthetizing the New Zealand white rabbits, the abdominal cavity was opened, the liver was lifted and placed on pre-weighed gauze, and a 6-mm diameter penetrating wound was created on the liver to induce bleeding. Subsequently, hemostatic materials from different groups were packed into the penetrating wound site. Blood loss was measured by weighing the hemostatic materials and gauze. During the experiment, compared to Group B, Group A showed a significant reduction in blood loss (Group A: 2.6 g, Group B: 4.5 g) and a significant reduction in hemostasis time (Group A: 35 s, Group B: 96 s). It is evident that the aforementioned polysaccharide-based sponge material can be applied for hemostasis in non-compressive penetrating injuries.

The polysaccharide-based sponge materials of other formulations in this invention can also be applied for hemostasis in non-compressive penetrating injuries.

Embodiments 16 to 22 illustrate the applications of specific formulations of polysaccharide-based two-component hydrogels in tissue repair, sealing of tissue fluid leakage, sealing of tissue air leakage, and hemostasis. These applications primarily utilize the tissue adhesion properties and hemostatic properties of polysaccharide-based two-component hydrogels. As described in Embodiments 12 to 14, the polysaccharide-based dual-component hydrogel Formulations 1-36 provided in the embodiments of the present invention all exhibit excellent tissue adhesion properties, hemostatic properties, and low swelling properties. Therefore, the polysaccharide-based dual-component hydrogel Formulations 1-36 provided in the embodiments of the invention can all be applied in tissue repair, sealing of tissue fluid leakage, sealing of tissue air leakage, hemostasis, and other applications.

Embodiments 23 to 34 provide specific formulations for polysaccharide-based membrane materials, polysaccharide-based powder materials, and polysaccharide-based sponge materials, along with their corresponding biomedical applications. Those skilled in the art may, based on the disclosure of this application, prepare the polysaccharide-based membrane materials, polysaccharide-based powder materials, and polysaccharide-based sponge materials using the polysaccharide-based two-component hydrogel Formulations 1-36 provided in the embodiments. Similarly, those skilled in the art may, based on the disclosure of this application, prepare polysaccharide-based membrane materials from the polysaccharide-based two-component hydrogel formulations 1-36 provided in the embodiments for use as sutureless wound closure or patches; or prepare polysaccharide-based powder materials from the polysaccharide-based dual-component hydrogel Formulations 1-36 provided in the embodiments for use as hemostatic agents, or as carriers for cells, factors, drug carriers; or, as needed, the polysaccharide-based sponge materials prepared from the polysaccharide-based dual-component hydrogel Formulations 1-36 provided in the embodiments may be used as wound dressing products or hemostatic products.

The above embodiments intend to facilitate the understanding and use of the invention. It is obvious that operators can easily make various modifications to these embodiments and the general principles described herein can be applied to other embodiments. Therefore, the invention is not limited to the embodiments described above. If the modifications and changes were not departing from the scope of this invention, they shall be under protection of this invention.

## Claims

1. A polysaccharide-based polymer crosslinker, **characterized in that** it is a polysaccharide-based polymer crosslinker modified with o-phthalaldehyde group, and the structure is shown in Formula 1:

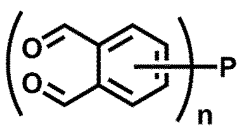

Formula 1

wherein, P is a natural polysaccharide macromolecule or a modifier or degradation thereof, and P is selected from one or more of hyaluronic acid, cellulose, cellulose derivatives, alginate, dextran, agarose, heparin, chondroitin sulfate, carrageenan, astragalus gum, xanthan gum, gellan gum, guar gum, gum arabic, acacia bean gum, starch, starch hydrolysate, or starch derivatives;

$$n \geq 2;$$

the o-phthalaldehyde group is connected by a covalent bond to P.

2. The polysaccharide-based polymer crosslinker according to claim 1, **characterized in that** the polysaccharide-based polymer crosslinker shown in Formula 1 is selected from one of the following structures:

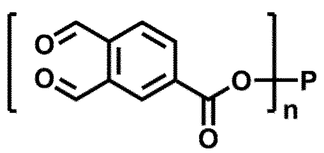

,

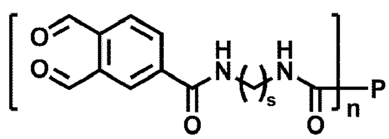

,

Formula 1-1　　Formula 1-2

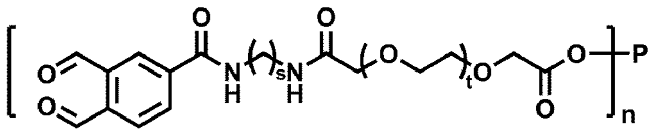,

Formula 1-3

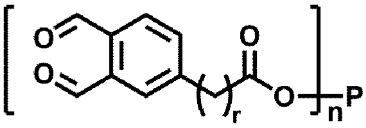, 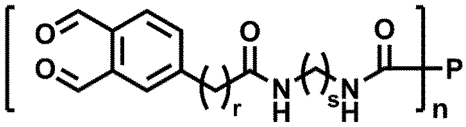,

Formula 1-4 Formula 1-5

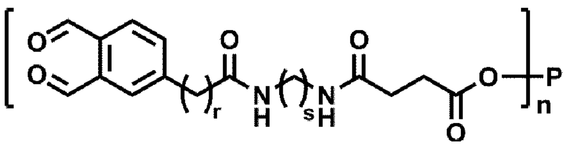,

Formula 1-6

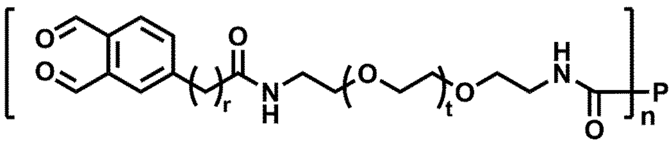,

Formula 1-7

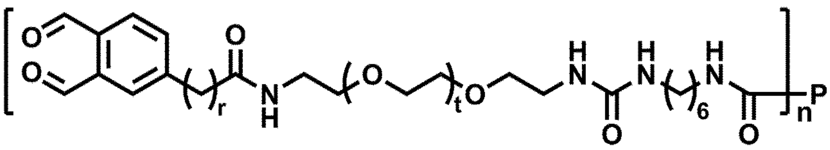,

Formula 1-8

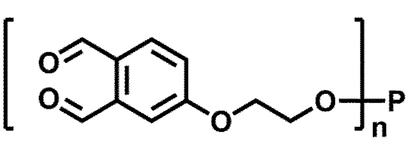, 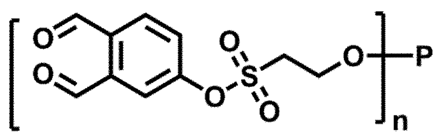,

Formula 1-9 Formula 1-10

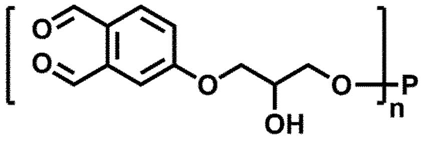

,

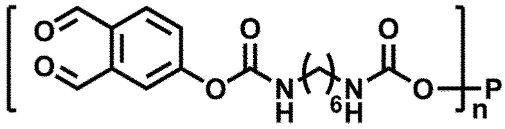

Formula 1-11 Formula 1-12

wherein, $1 \leq r \leq 20$, $1 \leq s \leq 20$, $1 \leq t \leq 50$, $n \geq 2$;
preferably, $1 \leq r \leq 6$, $1 \leq s \leq 10$, $1 \leq t \leq 30$, $n \geq 2$.

**3.** The polysaccharide-based polymer crosslinker according to claim 1, **characterized in that** P is selected from hyaluronic acid, cellulose derivatives, alginic acid, heparin, chondroitin sulphate, xanthan gum, gellan gum, starch or starch derivatives;

wherein, cellulose derivative is selected from methyl cellulose, carboxymethyl cellulose, hydroxyethyl cellulose, hydroxypropyl cellulose or hydroxypropylmethyl cellulose;
preferably, cellulose derivative is selected from carboxymethyl cellulose;
wherein, starch derivative is selected from oxidized starch, esterified starch, etherified starch or alkylated starch;
preferably, starch derivative is selected from carboxymethyl starch.

**4.** A method for preparing the polysaccharide-based polymer crosslinker of claim 1 or 2 or 3, **characterized in that** the aldehyde pre-protected o-phthalaldehyde precursor derivative is covalently bonded to the reactive group on the polysaccharide polymer to obtain the o-phthalaldehyde precursor derivative-modified polysaccharide polymer, and the o-phthalaldehyde precursor derivative-modified polysaccharide polymer is then de-protected to obtain the o-phthalaldehyde group-modified polysaccharide-based polymer crosslinker.

**5.** The method of preparing a polysaccharide-based polymer crosslinker according to claim 4, **characterized in that** the aldehyde pre-protected o-phthalaldehyde precursor derivative comprises an aldehyde pre-protected o-phthalaldehyde moiety and a substituent group R which can be connected to a reactive group on the polysaccharide polymer, with the structure shown in Formula 2:

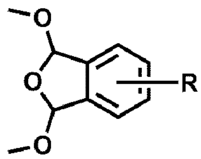

Formula 2

wherein, R is selected from a carboxyl substituent, a vinyl sulfone substituent, an epoxy substituent, a haloalkane substituent, an isocyanate substituent, or an amine substituent;
R is directly connected to the benzene ring or connected to the benzene ring through single or multiple alkylidene chains, alkoxy chains. These multiple alkylidene chains, alkoxy chains are connected to each other through ether, amide, ester, carbamate or urea bonds.

**6.** The method of preparing a polysaccharide-based polymer crosslinker according to claim 5, **characterized in that** aldehyde pre-protected o-phthalaldehyde precursor derivative is selected from one of the following compounds:

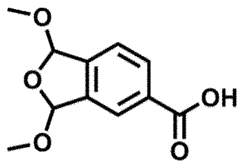
,
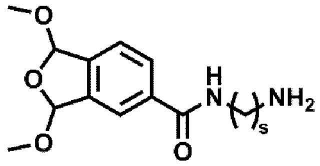
,

Compound 1 Compound 2

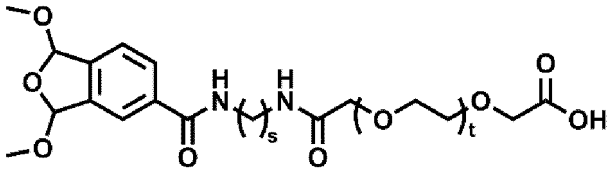
,
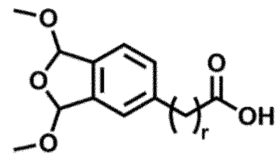
,

Compound 3 Compound 4

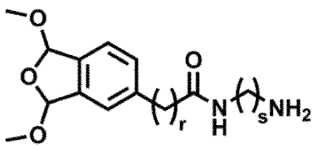
,
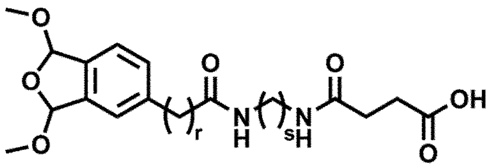
,

Compound 5 Compound 6

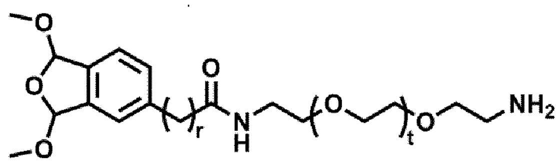

Compound 7

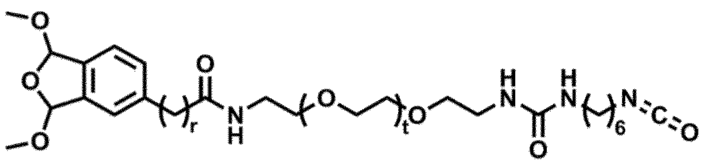

Compound 8

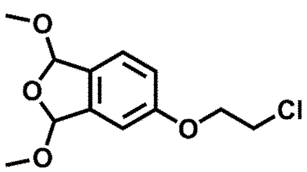
,
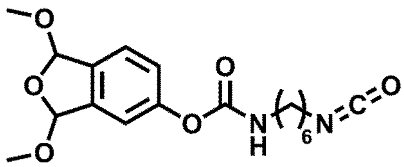
,

Compound 9 Compound 10

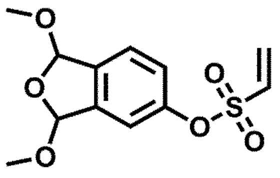 , 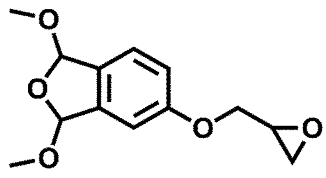

Compound 11 Compound 12

wherein, 1 ≤ r ≤ 20, 1 ≤ s ≤ 20, 1 ≤ t ≤ 50, n ≥ 2; preferably, 1 ≤ r ≤ 6, 1 ≤ s ≤ 10, 1 ≤ t ≤ 30, n >_ 2.

**7.** The method of preparing a polysaccharide-based polymer crosslinker according to claim 4, **characterized in that** reactive group of the polysaccharide polymer is an own reactive group of the polysaccharide polymer or a reactive group with reactive activity after the polysaccharide polymer has been modified or degraded;
preferably, the reactive group of the polysaccharide polymer is selected from hydroxyl group or carboxyl group.

**8.** The method of preparing a polysaccharide-based polymer crosslinker according to claim 4, **characterized in that** aldehyde pre-protected o-phthalaldehyde precursor derivative is covalently bonded to the active group on the polysaccharide polymer in a manner selected from ether bond, amide bond, ester bond, carbamate bond or urea bond connection, preferably ether bond, amide bond, ester bond, carbamate bond connection.

**9.** The method of preparing a polysaccharide-based polymer crosslinker according to claim 4, **characterized in that** when the active group of the polysaccharide polymer is selected from hydroxyl, the covalent bonding is based on ether bonding between vinyl sulfone substituents, epoxy substituents, or haloalkane substituents on the precursor derivatives of o-formaldehyde and hydroxyl groups on the polysaccharide polymer, on ester bonding between carboxylic acid substituents on o-phthalaldehyde precursor derivatives and hydroxyl groups on the polysaccharide polymer, on carbamate bonding between isocyanate substituents on o-phthalaldehyde precursor derivatives and hydroxyl groups on the polysaccharide polymer;
when the active group of the polysaccharide macromolecule is selected from a carboxyl group, the covalent bonding connection is based on an amide bonding connection between an amine group-like substituent on a precursor derivative of o-phthalaldehyde and a carboxyl group on the polysaccharide polymer.

**10.** The method of preparing a polysaccharide-based polymer crosslinker according to claim 4, **characterized in that** deprotecting the aldehyde group of the polysaccharide polymer modified by an o-phthalaldehyde precursor derivative means: deprotecting the aldehyde group attached to the polysaccharide polymer by a pre-protected o-phthalaldehyde functional group under acidic conditions to obtain the o-phthalaldehyde group-modified polysaccharide-based polymer crosslinker.

**11.** A method of preparing a polysaccharide-based dual-component hydrogel, **characterized in that** component A and component B are dissolved in a solvent to obtain a component A solution and a component B solution respectively, and the polysaccharide-based dual-component hydrogel is obtained by mixing the component A solution and the component B solution;

wherein, the component A is a polysaccharide-based polymer crosslinker modified with an o-phthalaldehyde group as claimed in any one of claims 1-3;
the component B is a water-soluble small molecule, a water-soluble synthetic macromolecule, a water-soluble natural macromolecule containing one or more of primary amine, biphenylamine, hydrazide, hydroxylamine or sulfhydryl groups, and the number of primary amine, biphenylamine, hydrazide, hydroxylamine or sulfhydryl functional groups contained on a single molecule is not less than 2;
the water-soluble natural macromolecules include proteins, nucleic acids and polysaccharides.

**12.** The method of preparing a polysaccharide-based dual-component hydrogel according to claim 11, **characterized in that** the component B is selected from one or more of the following substances: a polyethylene glycol derivative, a polyethyleneimine, a polyamino acid, a protein, a protein modifier, a protein degradation substance, a polysaccharide, a polysaccharide modifier, or a polysaccharide degradation substance; and that the substance selected for the component B contains in its molecular structure one or more groups of primary amines, biphenylamines, hydrazide, hydroxylamine or sulfhydryl group or groups, and the number of primary amine, biphenylamine, hydrazide, hydroxylamine or sulfhydryl functional groups contained on a single molecule is not less than 2;

preferably, the polyethylene glycol derivative is a polyethylene glycol derivative modified with a primary amine, a biphenylamine, a hydrazide, a hydroxylamine or a sulfhydryl group;
preferably, the proteins comprise collagen, serum proteins, fibrinogen and fibronectin, and the protein degradates comprise gelatin or peptides;
preferably, the polysaccharides, polysaccharide modifiers or polysaccharide degraders are selected from: chitosan and chitosan modifiers and chitosan degraders; tertiary amine, hydrazide, hydroxyl amine or sulfhydryl modified hyaluronic acid, alginic acid, chondroitin sulphate, heparin, cellulose, chitin and their respective modifiers and degraders.

**13.** The method of preparing a polysaccharide-based dual-component hydrogel according to claim 11, **characterized in that** the component B is selected from:
amine-modified polyethylene glycol derivatives; hydrazide-modified hyaluronic acid; collagen; serum protein; gelatin; polypeptides; polyamino acids; chitosan.

**14.** The method of preparing a polysaccharide-based dual-component hydrogel according to claim 11, **characterized in that** in the component A solution, the solid content of component A is 0.1-40 wt%, preferably 0.5-20 wt%, further preferably 0.5-10 wt%, and in the component B solution, the solid content of component B is 0.1-40 wt%, preferably 0.5-20 wt%, further preferably 0.5-10 wt%.

**15.** A polysaccharide-based dual-component hydrogel obtained on the basis of the preparation method described in claim 11 or 12 or 13 or 14.

**16.** A polysaccharide-based biomaterial, **characterized in that** it is selected from one of the following biomaterials:

a polysaccharide-based membrane material prepared by drying a polysaccharide-based dual-component hydrogel as claimed in claim 15;
a polysaccharide-based powder material prepared by further mechanical ball milling of said polysaccharide-based membrane material; or, a polysaccharide-based powder material prepared by mixing the component A solution, the component B solution of the polysaccharide-based dual-component hydrogel prepared in claim 11 by emulsification method, microfluidics method or mechanical fragmentation method, and then the microgel particles, which are further prepared by drying and sieving;
a polysaccharide-based sponge material containing a pore structure obtained by mixing a component A solution, a component B solution, and a pore-making agent of the polysaccharide-based dual-component hydrogel prepared in claim 11, and further produced by drying.

**17.** The polysaccharide-based biomaterial according to claim 16, **characterized in that** when the polysaccharide-based biomaterial is selected from polysaccharide-based sponge materials, the pore-making agent is an additive that causes pore structures to be created in the material and is selected from substances that readily decompose into gases, polymer microspheres, polyethylene glycol, polyvinylpyrrolidone, surfactants or water.

**18.** An application of the polysaccharide-based two-component hydrogel of claim 15, **characterized in that** it is selected from one of the following applications:

the application of said polysaccharide-based two-component hydrogel in the preparation of a tissue repair product;
the application of said polysaccharide-based two-component hydrogel in the preparation of a tissue fluid leakage sealing product;
the application of said polysaccharide-based two-component hydrogel in the preparation of tissue air leakage sealing products;
the application of said polysaccharide-based two-component hydrogel in the preparation of a hemostatic product;
wherein, The tissue repair includes skin repair, abdominal cavity wall repair; the tissue fluid leakage blockage includes pancreatic fluid leakage blockage, cerebrospinal fluid leakage blockage, intestinal leakage blockage, gastric leakage blockage; the tissue air leakage blockage includes lung parenchyma leakage blockage; the hemostasis includes liver hemostasis, kidney hemostasis, spleen hemostasis, pancreatic hemostasis, bone fraction hemostasis, arterial hemostasis, and cardiac hemostasis.

19. An application of the polysaccharide-based biomaterials of claim 16, **characterized in that** when the polysaccharide-based biomaterial is a polysaccharide-based membrane material, the application of the polysaccharide-based membrane material, selected from one of the following applications:

the application of the polysaccharide-based membrane material in the preparation of a suture-free closure product for surgical wounds;
the application of the polysaccharide-based membrane material in the preparation of a patch product;
when the polysaccharide-based biomaterial is a polysaccharide-based powder material, an application of the polysaccharide-based powder material, selected from one of the following applications:

the application of the polysaccharide-based powder material in the preparation of a hemostatic product;
the application of the polysaccharide-based powder material in the preparation of cell, factor, and drug carrier products;
the hemostasis includes minimally invasive surgical intraoperative hemostasis, liver hemostasis, kidney hemostasis, spleen hemostasis, pancreatic hemostasis, bone fraction hemostasis, arterial hemostasis, and cardiac hemostasis; and the cell, factor, and drug carriers include stem cell carriers, growth-promoting factor carriers, antimicrobial drug carriers, and inflammation-inhibiting drug carriers;
when the polysaccharide-based biomaterial is a polysaccharide-based sponge material, an application of the polysaccharide-based sponge material, selected from one of the following applications:

the application of the polysaccharide-based sponge material in the preparation of a wound dressing product;
the application of the polysaccharide-based sponge material in the preparation of a hemostatic product;
the hemostasis includes liver hemostasis, kidney hemostasis, spleen hemostasis, pancreatic hemostasis, bone fraction hemostasis, arterial hemostasis, and cardiac hemostasis, and hemostasis of a non-compressive penetrating wound.

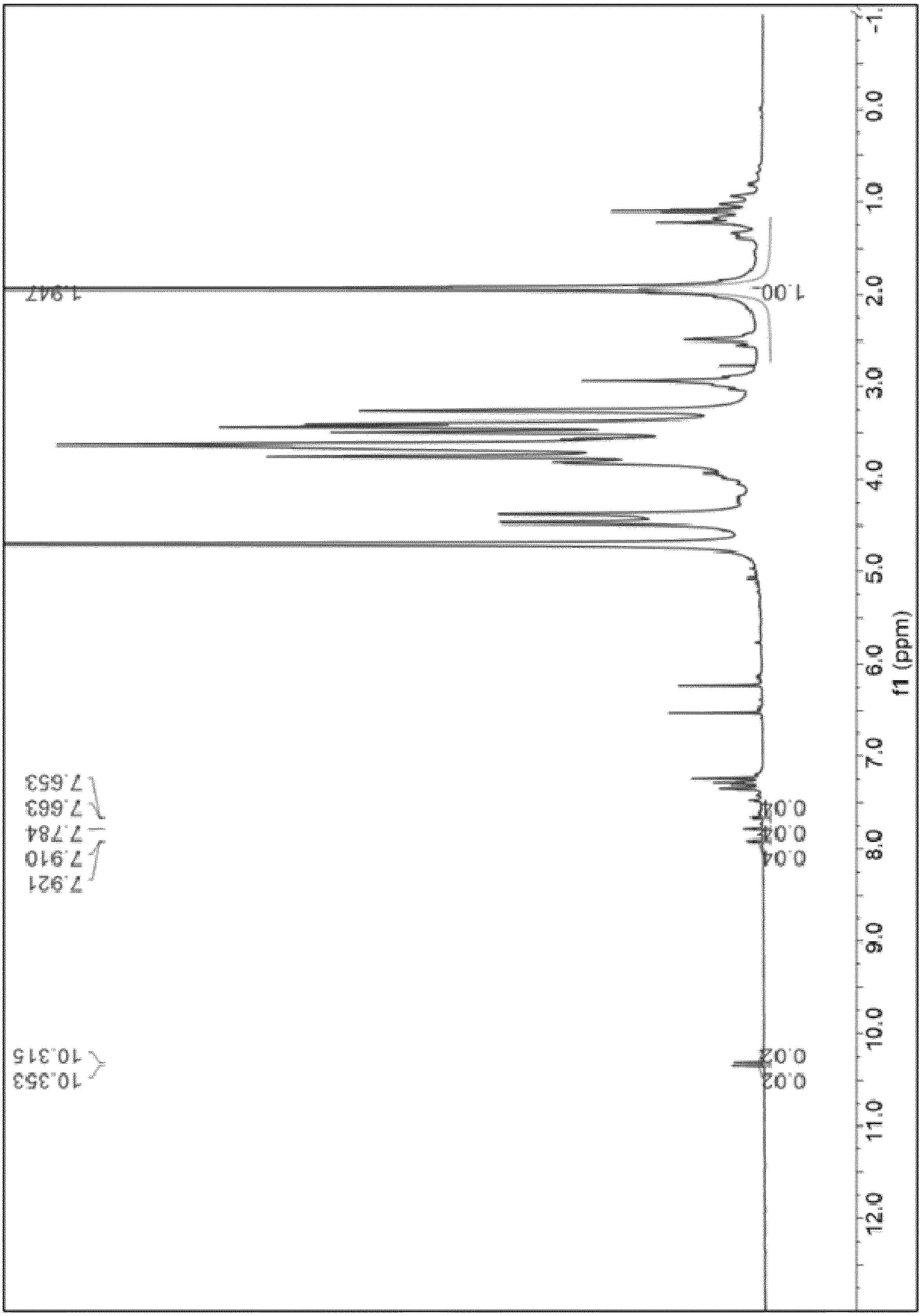


Fig. 1

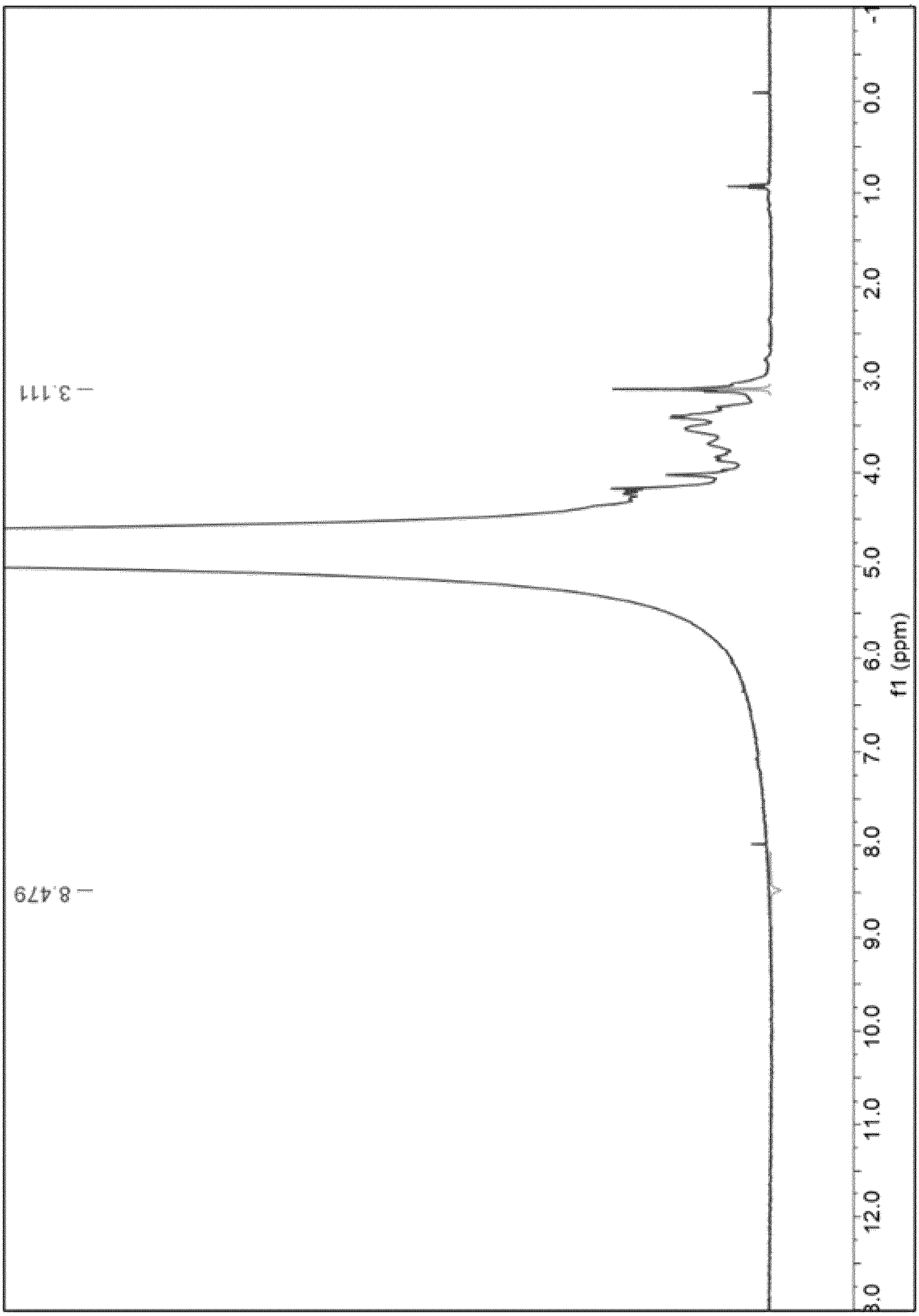


Fig. 2

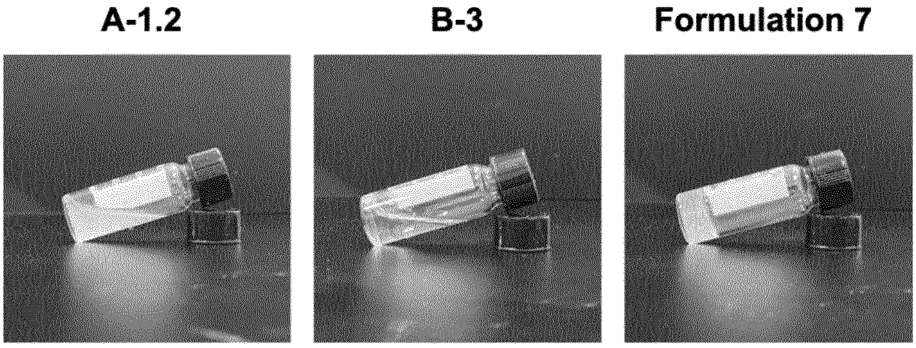


Fig. 3

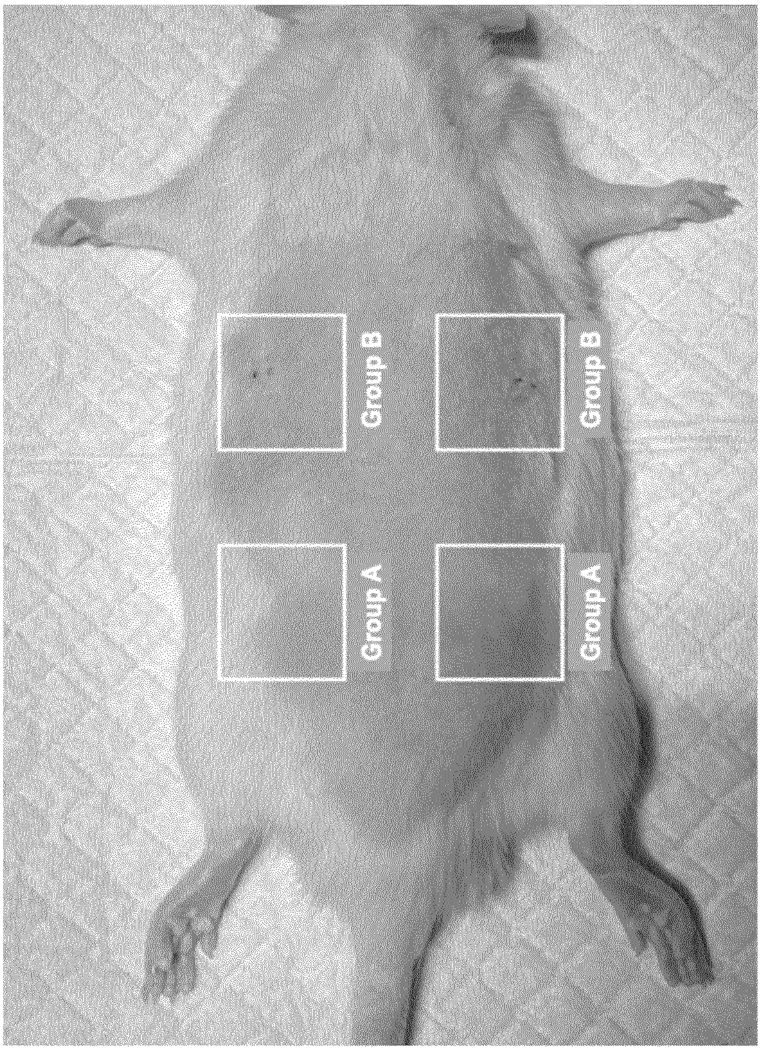


Fig. 4

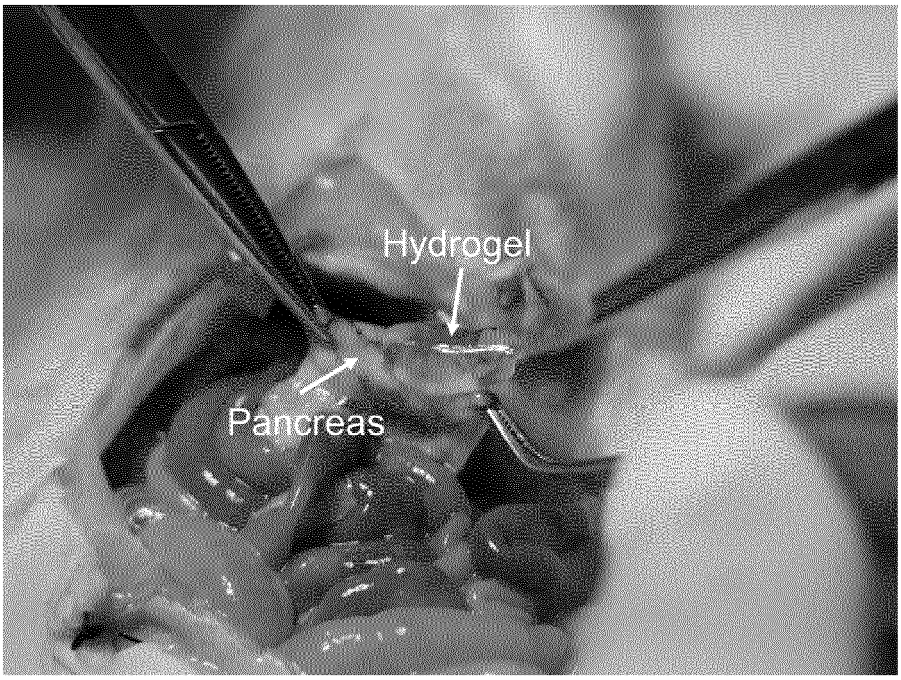


Fig. 5

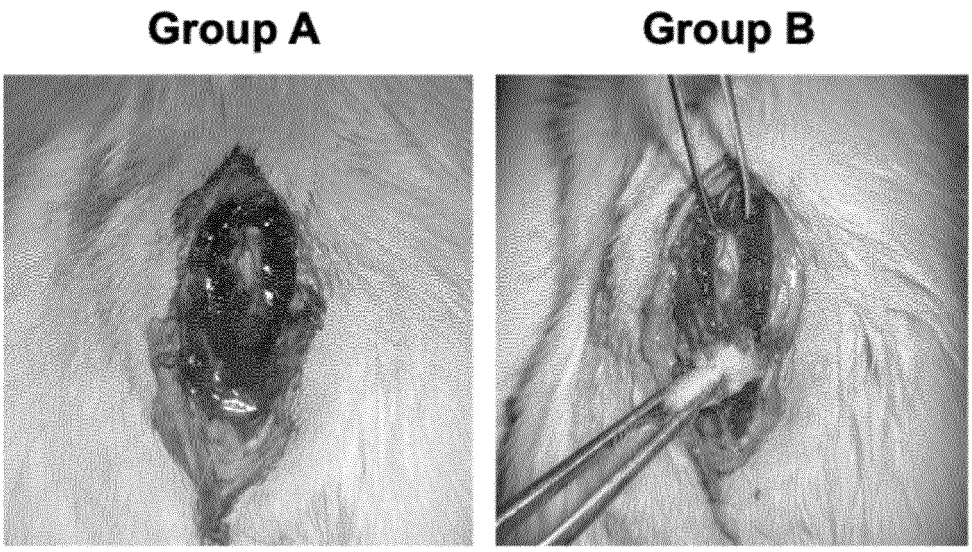


Fig. 6

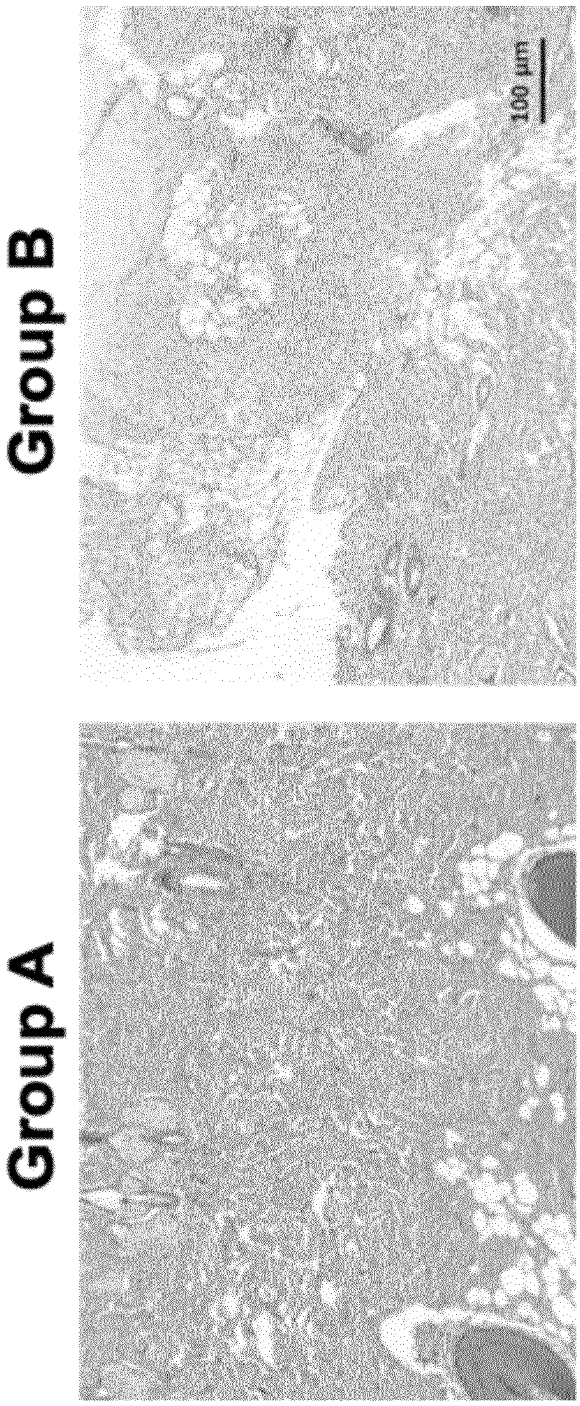


Fig. 7

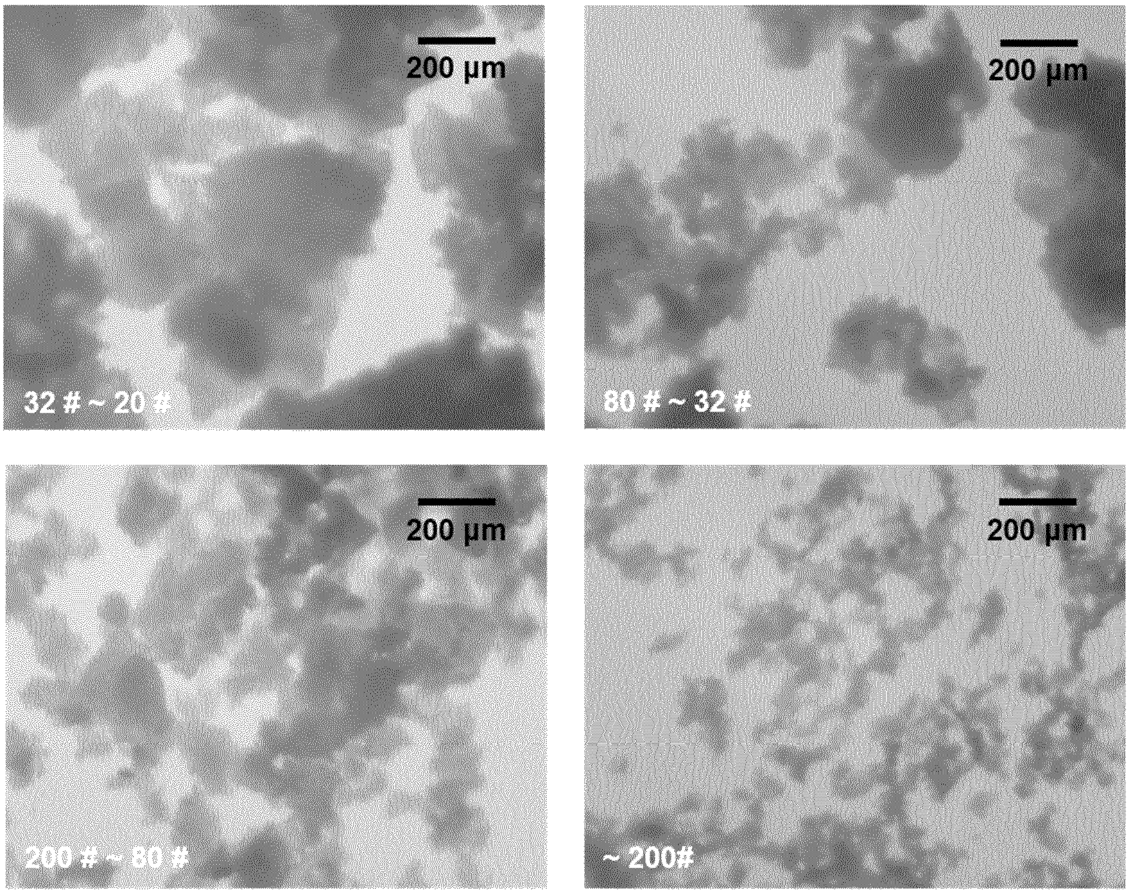


Fig. 8

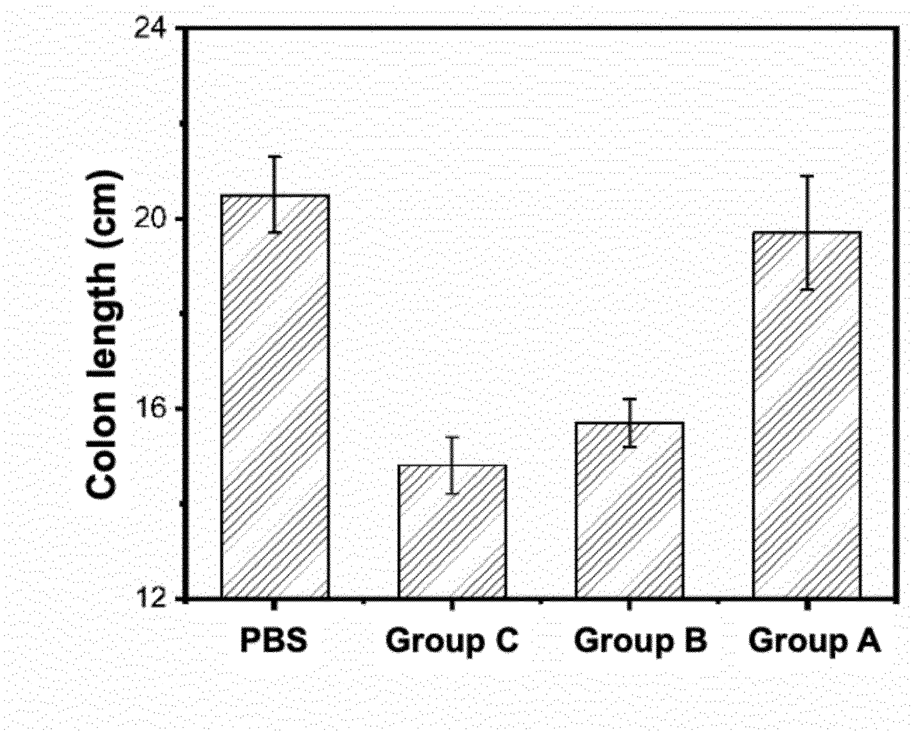
24
20
16
12
Colon length (cm)
PBS
Group C
Group B
Group A

Fig. 9

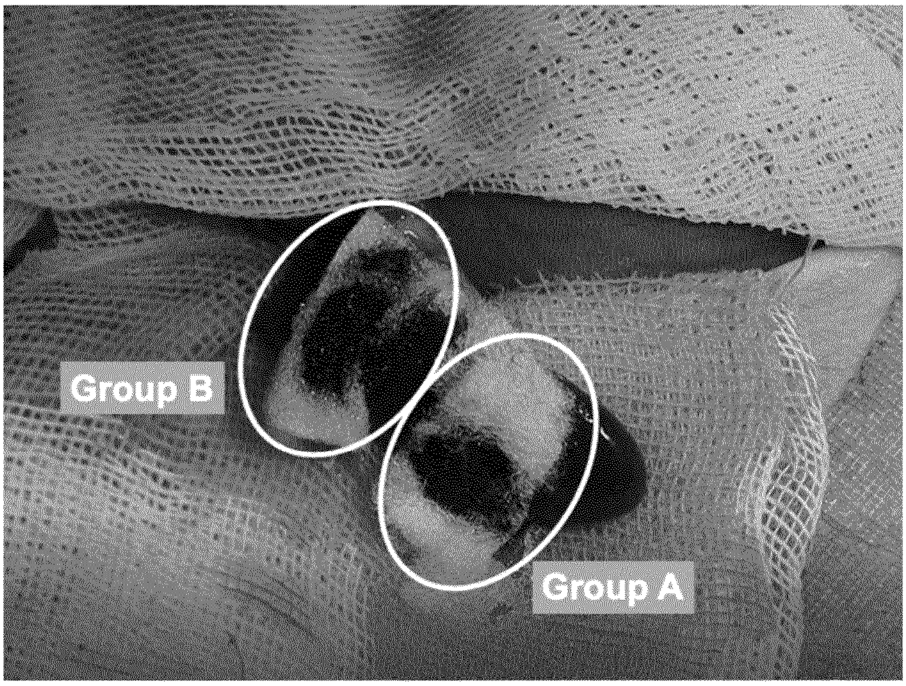
Group B
Group A

Fig. 10

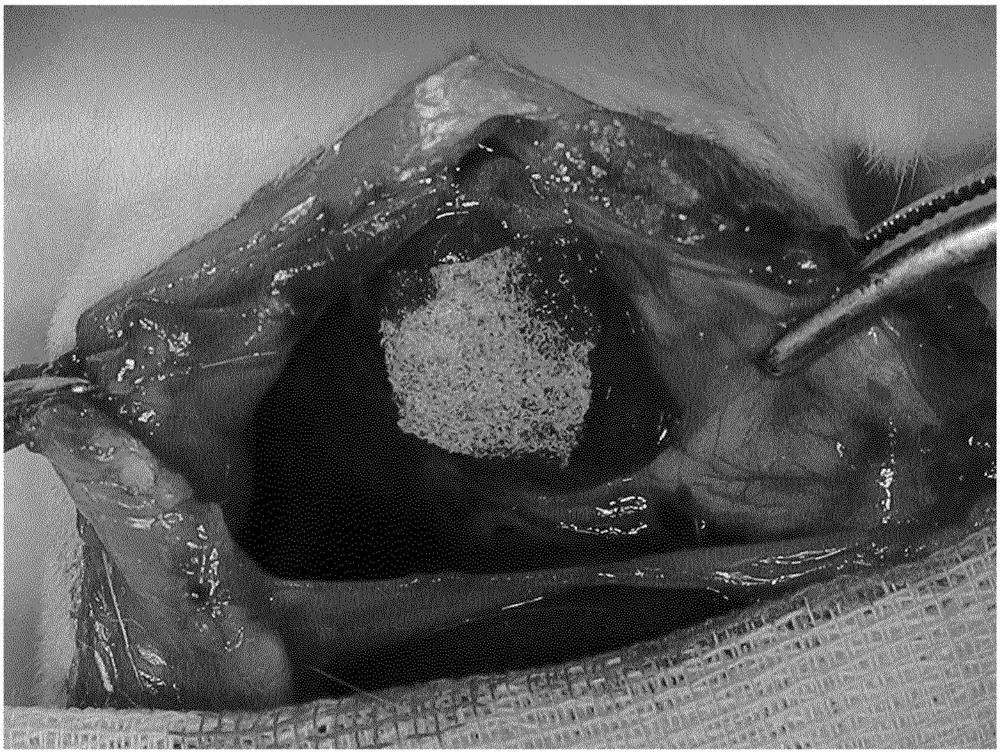

Fig. 11

# INTERNATIONAL SEARCH REPORT

International application No.
**PCT/CN2023/142840**

**A. CLASSIFICATION OF SUBJECT MATTER**

C08B37/08(2006.01)i； A61L27/52(2006.01)i

According to International Patent Classification (IPC) or to both national classification and IPC

**B. FIELDS SEARCHED**

Minimum documentation searched (classification system followed by classification symbols)

IPC: C08B, A61L

Documentation searched other than minimum documentation to the extent that such documents are included in the fields searched

Electronic data base consulted during the international search (name of data base and, where practicable, search terms used)

DWPI; VEN; CNTXT; CNKI; Elsevier; Caplus: 多糖, 交联剂, 邻苯二甲醛, 苯二醛, 透明质酸, 纤维素, 海藻酸, 葡聚糖, 琼脂糖, 肝素, 硫酸软骨素, 卡拉胶, 黄芪胶, 黄原胶, 结冷胶, 魔芋胶, 瓜尔胶, 阿拉伯胶, 刺槐豆胶, 淀粉, polysaccharide, crosslinker, phthalaldehyde, hyaluronic acid, cellulose, alginic acid, glucan, agarose, heparin, chondroitin sulfate, carrageenan, astragalus gum, xanthan gum, konjac gum, guar gum, arabic gum, locust bean gum, starch

**C. DOCUMENTS CONSIDERED TO BE RELEVANT**

| Category* | Citation of document, with indication, where appropriate, of the relevant passages | Relevant to claim No. |
|---|---|---|
| PX | CN 116284492 A (SHANGHAI JIAO TONG UNIVERSITY) 23 June 2023 (2023-06-23)<br>claims 1-19 | 1-19 |
| A | CN 114907580 A (SHANGHAI LINGJIU MEDICAL TECHNOLOGY CO., LTD.) 16 August 2022 (2022-08-16)<br>entire document | 1-19 |
| A | CN 114907558 A (SHANGHAI LINGJIU MEDICAL TECHNOLOGY CO., LTD.) 16 August 2022 (2022-08-16)<br>entire document | 1-19 |

☐ Further documents are listed in the continuation of Box C.　　☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **23 April 2024** | **24 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**
**Information on patent family members**

International application No.
**PCT/CN2023/142840**

| Patent document cited in search report | | | Publication date (day/month/year) | Patent family member(s) | | | Publication date (day/month/year) |
|---|---|---|---|---|---|---|---|
| CN | 116284492 | A | 23 June 2023 | None | | | |
| CN | 114907580 | A | 16 August 2022 | WO | 2022170681 | A1 | 18 August 2022 |
| | | | | US | 2023372582 | A1 | 23 November 2023 |
| CN | 114907558 | A | 16 August 2022 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 111440310 A
- CN 111440334 A
- CN 111574756 A
- CN 111621038 A
- CN 113509591 A
- CN 113694249 A
- CN 114767920 A
- CN 114907558 A
- CN 202010454896
- CN 202010455951


### Non-patent literature cited in the description


- **D THAVARAJAH** ; **P DE LACY** ; **R HUSSAIN** ; **RM REDFERN**. *Spine*, 2010, vol. 35, 25-26
- **ZHEN ZHANG** ; **CHAOLIANG HE** ; **YAN RONG** ; **HUI REN** ; **TIANRAN WANG** ; **ZHENG ZOU** ; **XUESI CHEN**. *National Science Review*, April 2021, vol. 8 (4)
- **CHUN LING TUNG** ; **CLARENCE T. T. WONG** ; **EVA YI MAN FUNG** ; **XUECHEN LI.** Synthesis of Compound 4: The synthesis process refers to the literature. *Org. Lett*, 2016, vol. 18 (11), 2600-2603